(19) Europäisches Patentamt
European Patent Office
Office européen des brevets

(11) Publication number: **0 399 949 B1**

(12) **EUROPEAN PATENT SPECIFICATION**

(45) Date of publication of patent specification: **12.04.95**

(51) Int. Cl.[6]: **C07F 9/38**, A61K 31/66

(21) Application number: **90810346.8**

(22) Date of filing: **08.05.90**

# (54) Substituted aminoalkylphosphinic acids.

(30) Priority: **13.05.89 GB 8911017**

(43) Date of publication of application:
**28.11.90 Bulletin 90/48**

(45) Publication of the grant of the patent:
**12.04.95 Bulletin 95/15**

(84) Designated Contracting States:
**AT BE CH DE DK ES FR GB GR IT LI LU NL SE**

(56) References cited:
**EP-A- 356 128**
**EP-A- 0 121 226**
**EP-A- 0 319 479**

(73) Proprietor: **CIBA-GEIGY AG**
**Klybeckstrasse 141**
**CH-4002 Basel (CH)**

(72) Inventor: **Hall, Roger Graham, Dr.**
**8 Uplands Road**
**Flixton,**
**Manchester M31 3PU (GB)**
Inventor: **Maier, Ludwig, Dr.**
**Suryhofweg 24**
**CH-4144 Arlesheim (CH)**
Inventor: **Fröstl, Wolfgang, Dr.**
**St. Johanns-Vorstadt 6/2**
**CH-4056 Basle (CH)**

(74) Representative: **Sharman, Thomas et al**
**CIBA-GEIGY PLC.**
**Patent Department,**
**Central Research,**
**Hulley Road**
**Macclesfield,**
**Cheshire SK10 2NX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The invention relates to P-substituted aminoalkylphosphinic acids of the formula

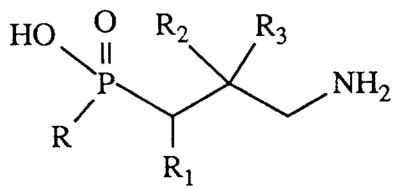

(I),

and pharmaceutically acceptable salts thereof, wherein R denotes an optionally fluorinated methyl group, $R_1$ denotes hydrogen, lower alkyl, lower alkoxy, hydroxy, halogen or a fluorinated methyl group and $R_2$ and $R_3$ denote hydrogen or $R_2$ denotes hydroxy, lower alkoxy or halogen and $R_3$ is hydrogen or $R_2$ and $R_3$ together represent an oxo group, with the exception of P-(3-aminopropyl)-P-methyl-phosphinic acid and acid addition salts, alkali metal salts and the magnesium salt thereof, for use in a method for the treatment of the human or animal body, to such methods of treatment, to the use of compounds of the formula I and of their pharmaceutically acceptable salts as medicaments or for the manufacture thereof, to pharmaceutical compositions containing the same and to compounds of the formula I *per se* with the exception of P-(3-amino-2-oxo-propyl)-P-methyl-phosphinic acid, racemic P-(3-amino-2-hydroxy-propyl)-P-methyl-phosphinic acid, P-(3-aminopropyl)-P-methyl-phosphinic acid and acid addition salts, alkali metal salts, the magnesium salt and the ammonium salt of P-(3-aminopropyl)-P-methyl-phosphinic acid, as well as to a process for the manufacture thereof.

Fluorinated methyl denotes fluoro-, difluoro- or trifluoromethyl.

Within the scope of the invention, there are to be understood by "lower" radicals and compounds, for example, those having up to and including 7, especially up to and including 4, carbon atoms. Also, the general terms have the following meanings:

Lower alkyl is, for example, $C_1$-$C_4$alkyl, such as methyl, ethyl, n-propyl or n-butyl, also isopropyl, isobutyl, secondary butyl or tertiary butyl, but may also be a $C_5$-$C_7$alkyl group, that is to say a pentyl, hexyl or heptyl group.

Lower alkoxy is, for example, $C_1$-$C_4$alkoxy, such as methoxy, ethoxy, n-propoxy or n-butoxy, also isopropoxy, isobutoxy, secondary butoxy or tertiary butoxy, but may also be a $C_5$-$C_7$alkoxy group, that is to say a pentoxy, hexoxy or heptoxy group.

Halogen is, for example, halogen of an atomic number up to and including 35, such as fluorine, chlorine or, less preferred, bromine.

The compounds of the formula I are of amphoteric nature and may be present in the form of internal salts. They also can form acid addition salts and salts with bases. Such salts are particularly pharmaceutically acceptable acid addition salts thereof, as well as pharmaceutically acceptable salts formed with bases. Suitable acids for the formation of acid addition salts are, for example, mineral acids such as hydrochloric, hydrobromic, sulphuric or phosphoric acid, or organic acids such as sulphonic acids, e.g. benzenesulphonic, p-toluenesulphonic or methanesulphonic acid, or carboxylic acids e.g. acetic, lactic, palmitic, stearic, malic, maleic, fumaric, tartaric, ascorbic or citric acid. Salts of compounds of the formula I with bases are, for example, alkali metal salts, e.g. sodium or potassium salts, or alkaline earth metal salts, e.g. calcium or magnesium salts, as well as ammonium salts, such as those with ammonia or organic amines, e.g. diethylamine, di(2-hydroxyethyl)-amine or tri-(2-hydroxyethyl)amine.

Depending on the presence of asymmetric carbon atoms, the compounds of formula I may be in the form of mixtures of isomers, particularly racemates, or in the form of pure isomers, especially optical antipodes.

The compounds disclaimed hereinbefore are known *per se*. P-(3-aminopropyl)-P-methyl-phosphinic acid and certain acid addition salts, alkali metal salts and the magnesium salt thereof are described as pharmaceutical agents in EP0356128. The sodium salt of this acid has been described in DE-OLS 2032712 [Chem. Abstracts 76,72656 k (1972)]. In this reference, however, the utility ascribed to the sodium and ammonium salts of P-(3-aminopropyl)-P-methyl-phosphinic acid is that of an intermediate for the manufacture of flame retardants or surfactants. There is no suggestion in DE-OLS 2032712 that P-(3-aminopropyl)-P-methyl-phosphinic acid or the salts thereof might have any pharmaceutical activity. The other disclaimed compounds, including the ammonium salt of P-(3-aminopropyl)-P-methyl-phosphinic acid, are novel as

pharmaceutical agents. P-(3-amino-2-hydroxy-propyl)-P-methyl phosphinic acid has been described, in its racemate form, in an article by J.G.Dingwall, "Phosphorus and Sulphur", Vol. 18, pages 353-356(1983). No direct utility is ascribed to this compound in the Dingwall article. P-(3-amino-2-oxo-propyl)-P-methyl-phosphinicacid is described in an article by Natchev, Tetrahedron, Vol. 44/20, pages 6455-6463, (1988). P-(3-amino-2-oxo-propyl)-P-methyl-phosphinic is said by Natchev to possess herbicidal activity.

The enantiomers of P-(3-amino-2-hydroxy-propyl)-P-methyl-phosphinic acid are novel and, as such, form part of the present invention.

The compounds of this invention have been found to have very strong affinities towards $GABA_B$ receptor sites, with inhibitory concentrations in the low nanomolar range. Specifically, they are $GABA_B$-agonists of high potency as can be demonstrated in vitro, for example, by their potentiation, in the low nanomolar range, of the stimulation of adenylate cyclase by noradrenaline in slices of rat cerebral cortex. *In vivo*, the compounds of the invention exhibit, in analogy to the known $GABA_B$-agonist $\beta$-(aminomethyl)-p-chlorohydrocinnamic acid (baclofen), muscle-relaxant activities as can be shown in the mouse and in the rat, for example, by means of the rotarod test. They also exhibit analgesic activities as can be shown in the phenyl-p-benzoquinone writhing syndrome of the mouse.

The compounds of the invention can be used as muscle relaxants, especially in spinal spasticity, multiple sclerosis and cerebral palsy, and as antispastics and analgesics in trigeminus neuralgia and in the drug-withdrawal syndrome.

Representative compounds of the invention have been found to be much more potent than baclofen in the rotarod test on rats and mice and to have a much longer duration of action. On the other hand, the known $GABA_B$-agonist 3-amino-propylphosphinic acid was inactive in either species up to very high doses.

Representative compounds of the invention have to be found to be much more active than baclofen in the phenyl-p-benzoquinone writhing syndrome of the mouse, too.

The aforementioned advantageous properties render the compositions of this invention of great value as specific therapeutic agents for mammals including man.

The invention relates in the first place to compounds of the formula I, wherein R denotes methyl, fluoromethyl, difluoromethyl or trifluoromethyl, $R_1$ denotes hydrogen, lower alkyl, lower alkoxy, hydroxy, halogen, fluoromethyl, difluoromethyl or trifluoromethyl and $R_2$ and $R_3$ denote hydrogen or $R_2$ denotes hydroxy, lower alkoxy or halogen and $R_3$ denotes hydrogen or $R_2$ and $R_3$ together represent oxo, and their pharmaceutically acceptable salts for use in a method for the treatment of the human or animal body, to pharmaceutical compositions containing the same and to compounds of the formula I *per se* and their salts, with the exception of the compounds hereinbefore disclaimed, as well as a process for the manufacture thereof.

The invention relates especially to compounds of the formula I, wherein R denotes methyl, fluoromethyl, difluoromethyl or trifluoromethyl, $R_1$ denotes hydrogen or $C_1$-$C_4$alkyl, such as methyl or ethyl, $R_2$ denotes hydrogen or hydroxy and $R_3$ denotes hydrogen or $R_2$ and $R_3$ together represent oxo, and their pharmaceutically acceptable salts for use in a method for the treatment of the human or animal body, to pharmaceutical compositions containing the same and to compounds of the formula I and their salts *per se* with the exception of the compounds hereinbefore disclaimed, as well as to a process for the manufacture thereof.

The invention relates also to compounds of the formula I, wherein R denotes methyl, fluoromethyl, difluoromethyl or trifluoromethyl, $R_1$ denotes hydroxy and $R_2$ and $R_3$ are hydrogen, and to their salts.

Subject to the foregoing disclaimers, the invention relates very especially to compounds of the formula I, wherein R denotes methyl, $R_1$ is hydrogen, and wherein $R_2$ represents hydrogen or hydroxy and $R_3$ is hydrogen or $R_2$ and $R_3$ together represent oxo, provided that, when $R_2$ denotes hydroxy, the C-atom it is attached to has S-configuration, in the free form, or in the form of an acid addition salt, to pharmaceutical compositions containing them and to a process for the manufacture thereof.

The invention relates specifically to the compounds of the formula I described in the examples, to their manufacture and/or use.

The invention relates very specifically to P-[3-amino-2(S)-hydroxy-propyl]-P-methyl-phosphinic acid in the free form and to pharmaceutical compositions containing the same or P-(3-amino-2-oxo-propyl)-P-methyl-phosphonic acid in the free form.

According to the present invention, there is also provided a process for the manufacture of compounds of formula I, characterised in that

a) in a compound of formula

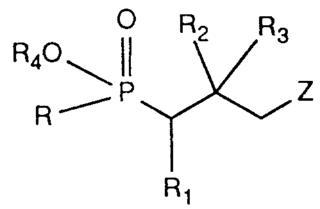

(II),

in which R, $R_1$, $R_2$ and $R_3$ have their previous significances, Z is $-NH_2$ and $R_4$ is a hydroxy-protective group $R_5$ or, when R is methyl and $R_1$, $R_2$ and $R_3$ are hydrogen, $R_4$ is an alkali metal or ammonium ion $R_6$, or Z is a protected or latent amino group $Z_0$, and $R_4$ is hydrogen or a hydroxy-protective group $R_5$, and wherein a carbonyl group formed by $R_2$ and $R_3$ together with the carbon atom to which they are attached may also be present in a temporarily protected form, any group $R_5$ or $R_6$ is replaced by hydrogen and/or any group $Z_0$ is converted into $-NH_2$ and/or, if $R_2$ and $R_3$ together with the carbon atom to which they are attached form a protected carbonyl group, such protecting group is removed; or

b) in a compound of the formula

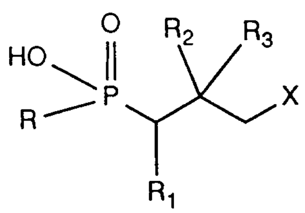

(III),

in which R, $R_1$, $R_2$ and $R_3$ have their previous significances and X is a group capable of being converted into the group of formula $-CH_2-NH_2$ (Ia), the group X is converted into the group; or

c) a compound of formula I' being identical to a corresponding compound of formula I apart from having one or more carbon-carbon multiple bond(s) is reduced to produce a compound of formula I wherein R has its previous significance, $R_1$ is hydrogen, lower alkyl or fluorinated methyl and $R_2$ and $R_3$ are hydrogen, and, if desired, a resulting compound is converted into another compound of the formula I, a resulting mixture of isomers is separated into the individual isomers and/or a resulting salt obtained in this process is converted into the free compound of the formula I or into another salt and/or, if desired, a resulting free compound of the formula I is converted into a salt to correspond to the above definition.

Protected hydroxy groups such as groups $-OR_5$ in starting materials of the formula II are, for example, etherified hydroxy groups, such as hydroxy groups etherified with an aliphatic, cycloaliphatic or araliphatic alcohol, e.g. with a $C_1$-$C_7$alkanol, a $C_1$-$C_7$-alkanoyloxy-$C_1$-$C_7$-alkanol, a cycloalkanol, or a $C_1$-$C_7$-alkanol substituted by one or two optionally substituted phenyl groups, or hydroxy groups etherified with an aliphatic silanol, e.g. with a tri-$C_1$-$C_7$alkylsilanol. As groups $R_5O$-, $C_1$-$C_7$alkoxy, e.g. $C_1$-$C_4$alkoxy, mono- or diphenyl-$C_1$-$C_7$-alkoxy, e.g. 1-phenyl- or 1,1-diphenyl-$C_1$-$C_4$-alkoxy, and tri-lower alkylsilyloxy, e.g. tri-$C_1$-$C_4$-alkyl-, such as trimethylsilyloxy, are especially preferred.

Protected amino groups such as groups $Z_0$ in starting materials of the formula II are, for example, acylamino groups such as $C_1$-$C_7$alkanoylamino, e.g. acetylamino, or phthalimido, $C_1$-$C_7$alkoxycarbonylamino groups unsubstituted or substituted by phenyl, e.g. benzyloxycarbonylamino or tert.-butoxycarbonylamino, or 1-aryl-$C_1$-$C_7$-alkylamino groups, e.g. benzylamino, or silylated amino groups, such as tri-$C_1$-$C_7$-alkylsilylamino or especially bis(tri-$C_1$-$C_7$-alkylsilyl)amino, e.g. bis(trimethylsilyl)amino. A latent amino group $Z_0$ may be e.g. nitro or azido.

Preferred compounds of formula II are those having the formula

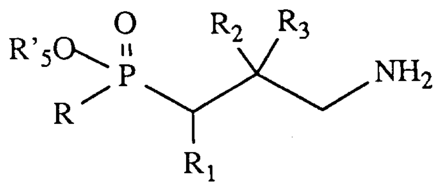

(IIa),

wherein R'$_5$ represents a hydroxy- protective group, for example, $C_1$-$C_4$alkyl or $C_1$-$C_4$-alkyl substituted by $C_1$-$C_7$alkanoyloxy or by one or two optionally substituted phenyl groups, such as 1-($C_2$-$C_7$alkanoyloxy)-$C_1$-$C_4$-alkyl, e.g. pivaloyloxymethyl or 1-phenyl- or 1,1-diphenyl-$C_1$-$C_4$alkyl e.g. benzyl, or having the formula

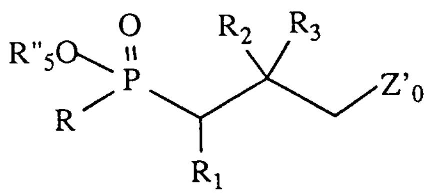

(IIb),

wherein R"$_5$ represents a hydroxy-protective group, for example, $C_1$-$C_4$-alkyl or $C_1$-$C_4$-alkyl substituted by one or two optionally substituted phenyl groups, such as 1-phenyl- or 1,1-diphenyl-$C_1$-$C_4$-alkyl e.g. benzyl, or a silyl group, such as tri-$C_1$-$C_4$-alkylsilyl, e.g. trimethylsilyl, and Z'$_0$ denotes, for example, $C_1$-$C_7$-alkanoylamino e.g. acetylamino, phthalimido or bis(silyl)amino, such as bis(tri-$C_1$-$C_4$-alkylsilyl)amino, e.g. bis(trimethylsilyl)amino, or having the formula

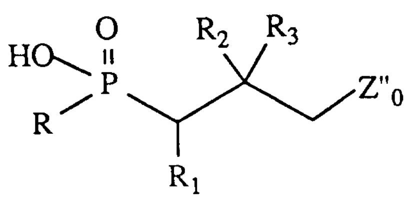

(IIc),

wherein Z"$_0$ denotes, for example, $C_1$-$C_7$-alkanoylamino, e.g. acetylamino, $C_1$-$C_4$alkoxycarbonylamino,e.g. tert.-butoxycarbonylamino, or phenyl-$C_1$-$C_4$-alkoxy-tert.-butoxycarbonylamino, or phenyl-$C_1$-$C_4$-alkoxycarbonylamino, or having the formula

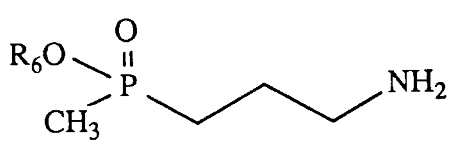

(IId),

wherein $R_6$ denotes an alkali metal or ammonium ion; and wherein in formulae IIa, IIb and IIc R, $R_1$, $R_2$ and $R_3$ have their previous significances.

The replacement of the protective group $R_5$, R'$_5$ or R"$_5$ in compounds of formula II, IIa or IIb by hydrogen may be effected by treatment with a suitable nucleophilic reagent, such as an alkali metal hydroxide, e.g. sodium or lithium hydroxide, an alkali metal halide, particularly bromide or iodide, such as lithium bromide or sodium iodide, thiourea or an alkali metal thiophenolate, such as sodium thiophenolate. The replacement reaction may be carried out in the absence or presence of a solvent and, if necessary, while cooling or heating, in a closed vessel and/or under an atmosphere of an inert gas.

When $R_5$, R'$_5$ or R"$_5$ denotes $C_1$-$C_4$alkyl substituted in 1-position by one or two phenyl groups, benzyl, the replacement of such a group in compounds of formula II, IIa or IIb by hydrogen may be effected by

hydrogenolysis in the presence of a metallic hydrogenation catalyst, or any other suitable procedure.

Alternatively, the replacement of the protective group, e.g. of a silyl group $R_5$ or $R''_5$ in compounds of formula II or IIb, of an alkyl group $R_5$, $R'_5$ or $R''_5$ in compounds of formula II, IIa or IIb, or of an alkali metal or ammonium ion $R_6$ in compounds of the formula II or IId by hydrogen may be effected by treatment with an acid under hydrolytic conditions, especially with a mineral acid such as hydrohalic acid, e.g. hydrochloric acid, which is used in diluted or concentrated aqueous form, or by treatment with an organic silyl halide such as trimethylsilyl iodide or bromide, followed by hydrolysis, if necessary. The reaction is preferably conducted at elevated temperature e.g. while refluxing the reaction mixture and, if necessary, using an organic diluent, in a closed vessel and/or under an atmosphere of an inert gas.

Protected amino groups $Z_0$, $Z'_0$ or $Z''_0$ in compounds of formula II, IIb or IIc or latent amino groups $Z_0$ in compounds of formula II may be converted into free amino according to known methods, which are selected according to the characteristics of the protected or latent amino group to be converted into amino, such as solvolytic or hydrogenolytic procedures, for example, hydrolysis in the presence of an acid or a base, acidolysis, e.g. treatment with trifluoroacetic acid, treatment with hydrazine, or hydrogenolysis in the presence of a metallic hydrogenation catalyst, or any other suitable procedure.

Depending on the groups involved, the replacement and conversion operations may be carried out in any sequence or simultaneously by methods which are well known per se.

It is preferred that all protecting groups are converted, $R_5$, $R'_5$, $R''_5$ or $R_6$ being converted to hydrogen, a carbonyl group, formed by $R_2$ and $R_3$ together with the carbon atom to which they are attached, in protected form being converted to a carbonyl group and $Z_0$, $Z'_0$ or $Z''_0$ being converted to $-NH_2$, in a single step, by treatment with an acid, preferably a hydrohalic acid, especially hydrochloric acid, under hydrolytic conditions.

The compounds of formula II may be prepared by various methods, for example according to the nature of the group X' in the formula V defined hereinafter, e.g. by reacting, in the presence of a basic catalyst or in the presence of agents forming free radicals, a compound of the formula

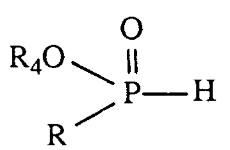

(IV),

in which R and $R_4$ have their previous significances, with a compound of formula

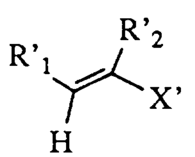

(V),

in which $R'_1$ is hydrogen, lower alkyl or fluorinated methyl, $R'_2$ is hydrogen or lower alkoxy and X' is a group X, which can be converted into the group Ia, or is a group $-CH_2-Z_0$ (VIa) in which $Z_0$ has its previous significance, in order to produce a compound of formula

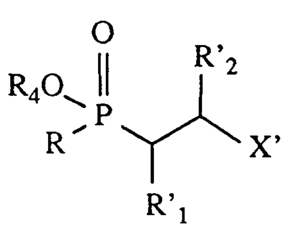

(VI),

wherein R, $R'_1$, $R'_2$, $R_4$ and X' have their previous significances, and which compound VI, when X' is a group VIIa, is identical to a compound II wherein $R_1$ is $R'_1$, $R_2$ is $R'_2$, $R_3$ is hydrogen and Z is $Z_0$; and then,

if a compound II is to be prepared wherein Z is amino, $R_1$ is $R'_1$, $R_2$ is $R'_2$ and $R_3$ is hydrogen, converting the group X' which is a group X into the group of formula Ia.

A group X is primarily cyano but may also represent carbamoyl or a group of the formula -CH=Y in which Y is a free or functionally modified oxo group such as a corresponding acetal or thioacetal group, including a corresponding cyclic group.

When, in the compound of formula V, X' is cyano or carbamoyl, then either a basic catalyst or a free radical catalyst may be employed. When, however, in the compounds of formula V, X' is e.g. a residue of formula $-CH_2-Z_0$ or -CH=Y, then a free radical catalyst is required.

A basic catalyst used in the first step may be e.g. an alkali metal $C_1$-$C_4$alkoxide, for example, a sodium or potassium $C_1$-$C_4$alkoxide, in particular sodium methoxide, sodium ethoxide or potassium tert.-butoxide, an alkali metal or alkaline earth metal fluoride such as potassium fluoride or caesium fluoride, or an alkali metal hydride, such as sodium hydride.

The reaction may be effected with or without the use of an added solvent. If a solvent is added, this is preferably an alcohol, in particular a $C_1$-$C_4$alkanol corresponding to the alkoxide used as basic catalyst. The reaction temperature may vary from 0 °C to the boiling point of any added solvent.

Agents forming free radicals are, for example, compounds convertible into free radicals by ionising or ultra-violet radiation, preferably peroxy compounds, such as inorganic peroxy compounds, e.g. hydrogen peroxide or ammonium persulphate, or organic peroxides, e.g. benzoyl peroxide or tert.-butyl peroxide, or organic azo compounds, e.g. azo-bis-iso-butyronitrile. Reactions involving free radical-forming agents may be conducted in the optional presence of a solvent and, if necessary, while cooling or heating, in a closed vessel and/or in an atmosphere of an inert gas.

The conversion of a group X into the group Ia is carried out according to known methods. Cyano and carbamoyl are converted into aminomethyl by reduction, cyano, for example, by hydrogenation in the presence of a suitable catalyst, e.g. Raney nickel, and of a solvent, such as ethanol, which may preferably contain ammonia, and carbamoyl, for example, by treatment with a suitable hydride reducing agent, such as borane in tetrahydrofuran. The conversion of a group -CH=Y into a group Ia is carried out by known deprotection followed by reductive amination procedures, e.g. treatment with sodium cyanoborohydride in the presence of ammonium acetate in a suitable solvent, such as dioxane, and while cooling, e.g. at about 0 °C.

The compounds of formula IV are either known or they may be,prepared e.g. by reaction of a compound of the formula R-P(Hal)$_2$ (IVa; Hal = halogen) with an alcohol $R_4OH$ in the presence of a tri-$C_1$-$C_7$-alkylamine. Specific examples of compounds of formula IV include:isopropyl(methyl)phosphonite and isobutyl(methyl)phosphonite.

Likewise, compounds of formula V are either known or can be obtained by methods which are well known.

Alternatively, in order to produce a compound of formula II wherein $R_4$ is $C_1$-$C_4$-alkyl or $C_1$-$C_4$-alkyl substituted by one or two optionally substituted phenyl groups and $R_1$ is $R'_1$, a compound of the formula

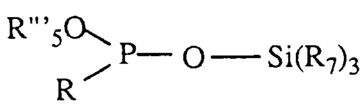 (VII),

in which R has the meaning indicated, $R'''_5$ is $C_1$-$C_4$alkyl or $C_1$-$C_4$alkyl substituted by one or two optionally substituted phenyl residues and each $R_7$, independently, is $C_1$-$C_7$alkyl, preferably $C_1$-$C_4$alkyl, particularly methyl, the groups $R'''_5$ and $R_7$ being the same or different, can be reacted with a compound of the formulae

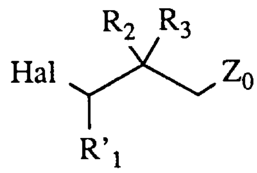

(VIIIa),

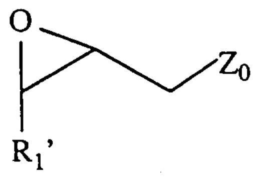

(VIIIb),

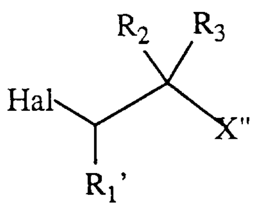

(VIIIc),

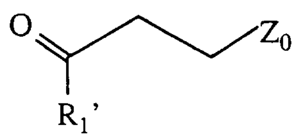

'IId),

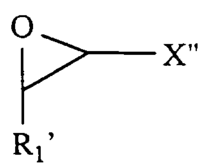

(VIIIe) or

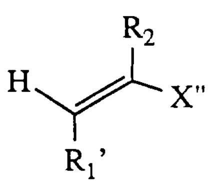

(V'),

in which $R_2$, $R_3$ and $Z_0$ have their previous significances, hydroxy $R_2$ or oxo $R_2 + R_3$ being present in a temporarily protected form, $R_1'$ is hydrogen, lower alkyl or a fluorinated methyl group, X" is primarily cyano or a group of the formula -CH=Y in which Y has its previous significance, which groups are subsequently converted into the group Ia, and Hal stands for halogen, such as iodo, bromo or chloro.

The reaction with an epoxide of formulae VIIIb or VIIIe is advantageously carried out in the presence of a mild Lewis acid, such as anhydrous zinc chloride, whilst the reaction with a halide of formula VIIIa or VIIIc is preferably carried out under the conditions of the Arbusov method, e.g. at a reaction temperature ranging from room temperature to 200 °C, e.g. to 160 °C, while removing the trialkyl silyl halide formed in the reaction.

In a modification of the reaction between a compound of formula VII and a compound of formula VIIIb, a compound of formula II wherein $R_1$ is $R'_1$, $R_2$ is hydroxy, $R_3$ is hydrogen, $R_4$ is $R'''_5$ and Z is $Z_0$ may be produced by reacting a compound of formula VII with a compound of formula

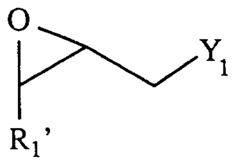

(VIIIf),

in which $R_1$' is hydrogen, lower alkyl or a fluorinated methyl group and $Y_1$ is a leaving group, for example, a reactive esterified hydroxy group, e.g. an arylsulphonyloxy group such as a tosyl group, and in which compound of formula VIIIf may be in racemic form or in the form of a single optically active isomer, to produce a compound of formula

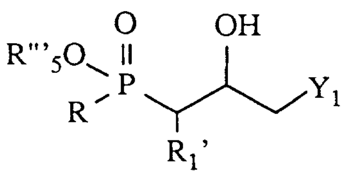

(IX),

in which R, R'$_1$ R'''$_5$ and $Y_1$ have their previous significances, and converting $Y_1$ into a group $Z_0$, e.g. by reaction with sodium azide.

Compounds of formula II may also be prepared starting from and N-protecting a compound of formula

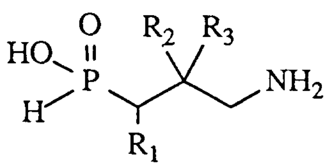

(X),

wherein $R_1$, $R_2$ and $R_3$ have their previous significances, to give a compound of formula

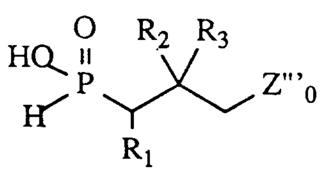

(XI),

wherein $R_1$, $R_2$ and $R_3$ have their previous significances and Z'''$_0$ is Z'$_0$ or Z''$_0$,and, subsequently, protecting also the (acid) hydroxyl group in the compound of formula XI to produce a compound of formula

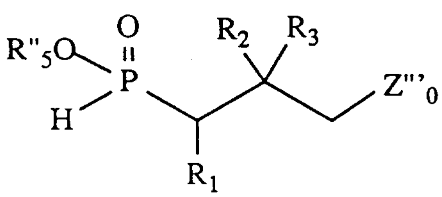

(XII),

wherein $R_1$, $R_2$, $R_3$, R''$_5$ and Z'''$_0$ have their previous significances.

Alternatively, in a preferred embodiment of process variant a), the starting material of formula X can be reacted with a silylating agent, such as a hexa-$C_1$-$C_7$-alkyldisilazane or a tri-$C_1$-$C_7$-alkyl halogenosilane, e.g. with hexamethyldisilazane or trimethylchlorosilane, in the presence of triethylamine, to produce a compound of formula

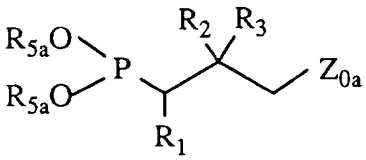

(XII'),

wherein $R_1$, $R_2$ and $R_3$ have their previous significances, $R_{5a}$ denotes tri-$C_1$-$C_7$-alkylsilyl, e.g. trimethylsilyl, and $Z_{0a}$ denotes tri-$C_1$-$C_7$-alkylsilylamino, such as trimethylsilylamino.

The intermediate of the formula XII or XII' is then reacted with a compound capable of converting the

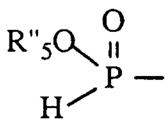

or

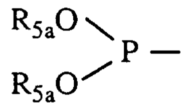

group into a

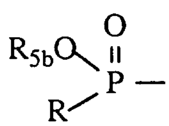

group , wherein R has its previous significance and $R_{5b}$ is $R''_5$ or $R_{5a}$, to produce the corresponding compound of formula II, in which $R_4$ is $R_{5b}$ and Z is $Z_{0a}$ or $Z'''_0$. Thus, the intermediate XII or XII' may be reacted with a compound of the formula R-$Y_2$ (XII''), wherein $Y_2$ is a reactive esterified hydroxy group, e.g. a halogen atom or a sulphonyloxy group such as p-toluenesulphonyloxy, for example, with methyliodide, fluoromethyl iodide, difluoromethyl iodide or trifluoromethyl iodide.

Most starting materials of formula X and their production have been described in US-Patent No. 4656298. Novel compounds of the formula X can be prepared in an analogous manner.

In another preferred embodiment of process variant a), a compound of the formula

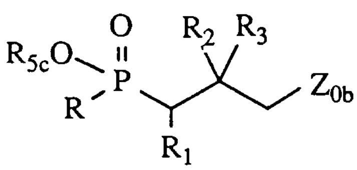

(IIb'),

wherein R, $R_1$, $R_2$ and $R_3$ have their previous significances, $R_{5c}$ is tri-$C_1$-$C_7$-alkylsilyl and $Z_{0b}$ is $Z_{0a}$ or $Z'''_0$ which compound IIb' may be prepared, for example, in a manner analogous to that shown in the reaction sequence

X---->XI---->XII---->IIb' or
X---->XII'---->IIb',

is subjected to basic or acidic hydrolysis to produce the corresponding compound II, wherein $R_4$ is hydrogen and Z is $Z_{0b}$. Advantageously, a compound IIb', wherein $R_{5c}$ denotes tri-$C_1$-$C_7$-alkylsilyl, $Z_{0b}$ denotes tri-($C_1$-$C_7$-alkylsilyl)amino and R, $R_1$, $R_2$ and $R_3$ have their previous significances, is formed *in situ* by reacting a compound of the formula X with a silylating agent and subsequently, preferably under basic conditions, with a compound XII'' and deprotected according to the invention, when worked up under protic, e.g. aqueous/alcoholic conditions.

Compounds of the formula II, wherein $R_2$ denotes hydroxy and $R_3$ represents hydrogen or $R_2$ and $R_3$ together denote oxo, may be produced by reacting a compound having the formula

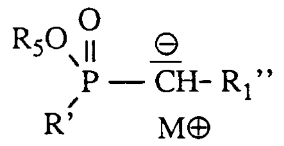

(XIII)

in the form of the salt of the formula

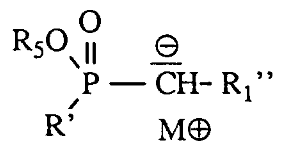

(XIII'),

wherein $R_5$ has its previous significance, either R' is optionally fluorinated methyl and $R_1$'' is hydrogen or fluorinated methyl, or R' is trifluoromethyl and $R_1$'' is lower alkyl, and M is an alkali metal, alkaline earth metal or transition metal, preferably lithium, sodium or potassium, calcium, zinc or tin, with a compound having the formula

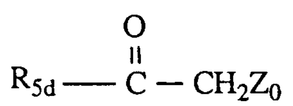

(XIV),

wherein $R_{5d}$ denotes etherified hydroxy such as specified for $R_5$ , halogeno, such as chloro or bromo, or hydrogen and $Z_0$ has its previous significance, to produce a compound having the formula II wherein $R_4$ is $R_5$, R is R', Z is $Z_0$, $R_1$ is $R'_1$, $R_2$ is hydroxy and $R_3$ denotes hydrogen or $R_2$ and $R_3$ together denote oxo.

The conversion of the group X into a group of formula $-CH_2-NH_2$ according to process variant b) may be effected by any of the methods described hereinbefore, e.g. by a variation of the conversion of compounds of formula VI into compounds of formula II. The reaction is carried out according to known methods, in the absence or presence of a solvent, which may also serve as a reagent, if necessary, while cooling or heating, in a closed vessel and/or in the atmosphere of an inert gas.

The starting material of the formula III may be prepared, for example, from compounds of a type similar to those of the formula VI, wherein $R_4$ is a group $R_5$ having its previous significance, by converting the group $R_5O$- into hydroxy, the reaction being carried out according to the previously described procedure, e.g. by acidic hydrolysis, such as by treatment with an aqueous mineral acid, e.g. hydrochloric acid, or by treatment with a nucleophilic reagent.

In process variant c), a compound of formula I' may have its unsaturation 1) within a substituent $R''_1$ corresponding after the reduction to the substituent $R_1$ in the end product of the formula I; or 2) between the carbon atom carrying the substituent $R_1$ and the carbon atom carrying the substituents $R_2$ and $R_3$ in the end product of the formula I. In the former case, the compound of formula I' will have the formula

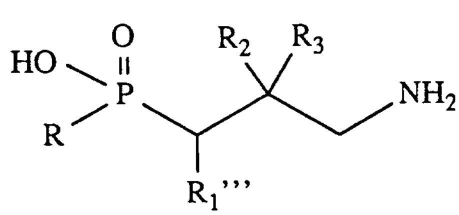

(I"),

in which R, $R_2$ and $R_3$ have their previous significances and $R_1$''' is $C_2$-$C_7$-alkenyl or $C_2$-$C_7$-alkynyl. In the latter case, the compound of the formula I' will have the formula

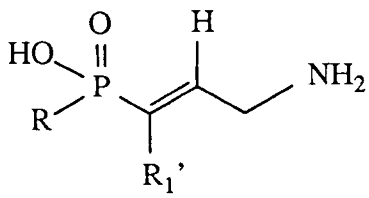

(I''')

wherein R has its previous significance and $R_1$' is hydrogen, lower alkyl or a fluorinated methyl group.

The reduction may be effected by any suitable reducing agent, such as hydrogen in the presence of a catalyst for the reduction of aliphatic multiple bonds e.g. palladium on charcoal, in the presence or absence of a solvent and at room temperature or elevated temperature.

The unsaturated compounds of formula I' may be produced according to any of the methods described herein for the manufacture of compounds of formula I, starting from corresponding unsaturated starring materials. The above-mentioned reactions are carried out according to standard methods, in the presence or absence of diluents, preferably such as are inert to the reagents and are solvents therefore, of catalysts, condensing or said other agents, respectively, and/or of inert atmospheres, at low temperatures, room temperature or elevated temperatures, preferably near the boiling point of the solvents used, and at atmospheric or super-atmospheric pressure.

Compounds of the formula I obtainable according to the process of the invention may be interconverted into one another.

Thus, compounds of formula I, wherein $R_1$ and/or $R_2$ denotes hydroxy, can be converted into the corresponding hydroxy-free compounds, for example, by reacting with thiocarbonyldiimidazole and treating the resulting imidazolylthiourethane in the presence of a radical-initiator, such as azo-bis-isobutyronitrile, with a tri-$C_1$-$C_7$alkylstannane, e.g. with $(C_4H_9)_3SnH$, for example in benzene at 60 to 80 °C.

Compounds of formula I, wherein $R_2$ and $R_3$, together with the carbon atom to which they are both attached, form a carbonyl group, may be converted into compounds in which $R_2$ is hydroxy and $R_3$ is hydrogen, by known reductive methods and *vice versa*, compounds of the formula I, wherein $R_2$ is hydroxy and $R_3$ is hydrogen, may be converted by known oxidative methods into the corresponding compounds I, wherein $R_2 + R_3$ are oxo..

The invention further includes any variant of the present processes, in which an intermediate product obtainable at any stage thereof is used as starting material and the remaining steps are carried out, or in which the starting materials are formed under the reaction conditions, or in which the reaction components are used in the form of their salts and/or racemates or optically pure antipodes.

Whenever desirable, the above processes are carried out after first suitably protecting any potentially interfering reactive functional groups, e.g. as illustrated herein. Advantageously, those starting materials should be used in the reactions described hereinbefore that lead to the formation of those compounds indicated above as being preferred. The invention relates also to novel starting materials and processes for their manufacture.

Depending on the choice of starting materials and methods, the new compounds may be in the form of one of the possible isomers, for example, as diastereomers, as optical isomers (antipodes), as racemates, or as mixtures thereof. If diastereomeric mixtures of the above compounds or intermediates are obtained, these can be separated into the single racemic or optically active isomers by methods in themselves known, e.g. by fractional distillation, crystallisation or chromatography. The racemic products of formula I or basic intermediates can be resolved into the optical antipodes, for example, by separation of diastereomeric salts thereof, e.g. by the fractional crystallisation of their (D)- or (L)-(tartrate, dibenzoyltartrate, mandelate or camphorsulphonate) salts. Advantageously, the more active of the antipodes of the compounds of this invention is isolated.

Furthermore, the compounds of the invention are either obtained in the free form, i.e. in the form of an internal salt ("Zwitterion" form), or as acid addition salts or salts with bases. For example, any resulting free compound can be converted into a corresponding acid addition salt, preferably with the use of a pharmaceutically acceptable acid or anion exchange preparation, or into a salt with bases by treatment of the free compounds with bases or suitable cation exchange techniques, or resulting salts can be converted into the corresponding free compounds, for example the acid addition salts, with the use of a stronger base, such as a metal or ammonium hydroxide, or any basic salt, e.g., an alkali metal hydroxide or carbonate, or a cation exchange preparation and the salts with bases by treatment with suitable acidic reagents.

These or other salts, for example the picrates, can also be used for purification of the compounds obtained; the compounds are then first converted into salts. In view of the close relationship between the

free compounds and the compounds in the form of their salts, whenever a compound is referred to in this context, a corresponding salt is also intended, provided such is possible or appropriate under the circumstances and the term "salts" shall, if desired also include the free compounds, where appropriate according to meaning and purpose. The compounds, including their salts, may also be obtained in the form of their hydrates, or include other solvents used for the crystallisation.

The pharmaceutical compositions according to the invention which contain compounds of the formula I or pharmaceutically acceptable salts thereof, are intended for enteral, such as oral or rectal, as well as parenteral administration and contain the pharmacologically active ingredient alone or in admixture to customary pharmaceutically acceptable carriers.

The pharmaceutical compositions of the invention contain, for example, from approximately 10% to 80%, preferably from approximately 20% to 60%, of the active ingredient. Pharmaceutical compositions according to the invention intended for enteral and parenteral administration are, for example, pharmaceutical compositions in dose unit form, such as dragées, tablets, capsules or suppositories, and also ampoules for injection. They are manufactured in a manner known *per se*, by means of conventional mixing, granulating, confectioning, dissolving or lyophilisating processes. For example, pharmaceutical compositions for oral administration can be obtained by combining the active ingredient with solid carriers, if desired granulating the resulting mixture and processing the mixture or granulate obtained, if desired or necessary after addition of suitable adjuncts, into tablets, tablet cores, dragées or capsules.

The present invention also relates to the use of the compounds of the invention for the preparation of pharmaceutical compositions, especially pharmaceutical compositions having selective $GABA_B$ agonistic activity which can be used in the treatment of spinal spasticity, multiple sclerosis, cerebral palsy, trigeminus neuralgia, drug withdrawal syndromes and/or conditions of pain.

These preparations may be used especially in the above-mentioned indications, if they are administered orally or parenterally, such as intravenously, intramuscularly or subcutaneously. The necessary dose depends on the particular disorder to be treated, its severity and the duration of therapy. The number and quantity of the individual doses and also the administration scheme is best determined on the basis of an individual examination of the host concerned, these methods being known to those skilled in the art. As a rule, however, a therapeutically active quantity of a compound of this invention is in the dosage range of about 0.1 to 10 mg/kg body weight per day. The pharmaceutical preparations are manufactured according to known methods, using standard auxiliary substances.

The following Examples further illustrate the present invention. Temperatures are given in degrees centigrade; pressures in mbar.

Example 1: A solution of 10.0 g of isobutyl P-(3-aminopropyl)-P-methyl-phosphinate in 60 ml of 36% aqueous hydrochloric acid is heated to reflux for a period of 15 hours. The reaction mixture is then allowed to cool to room temperature, concentrated under reduced pressure, and co-evaporated twice with 50 ml of water. The crude material is dissolved in water, washed with ether and the aqueous layer evaporated to dryness. The crude product is dissolved in 50 ml of methanol, 1-2 ml of propylene oxide are added and the mixture is stirred until the precipitated solid is free of halogen. The solid is filtered and dried to give P-(3-aminopropyl)-P-methyl-phospinic acid, m.p. 270-278°, $^{31}$P-NMR spectrum: $\delta = +42.1$ ppm ($D_2O$).

The starting material may be prepared as follows:

A solution of 15.0 g of isobutyl P-methylphosphonite and 5.3 g of acrylonitrile in 50 ml of dry ethanol is added to a stirred mixture of 0.5 g of sodium (50% dispersion in oil) in 25 ml of ethanol, at 0°C, under an atmosphere of nitrogen. The reaction mixture is allowed to warm to room temperature, and stirred for 4 hours. 1 ml of glacial acetic acid is added and the mixture is concentrated under reduced pressure. The resulting crude product is dissolved in 50 ml of ethyl acetate, washed twice with 20 ml of water, and the organic extract is dried over magnesium sulphate, and then concentrated under reduced pressure. The crude product is distilled to give isobutyl P-(2-cyanoethyl)-P-methyl-phosphinate, b.p. 140°/0.2mbar, $^{31}$P-NMR spectrum: $\delta = +50.5$ ppm ($CDCl_3$).

A solution of 20.0 g of isobutyl P-(2-cyanoethyl)-P-methyl-phosphinate in 200 ml of ethanol is added to 230.0 g of an 8% solution of ammonia in ethanol. To this are added 15 ml of Raney nickel slurry and the resulting mixture is hydrogenated at 1 bar until hydrogen uptake ceases. The mixture is then filtered and the filtrate is concentrated under reduced pressure. The crude product is distilled under reduced pressure to give isobutyl P-(3-aminopropyl)-P-methyl-phosphinate, b.p. 130°/0.01 mbar, $^{31}$P-NMR spectrum: $\delta = +57.6$ ppm ($CDCl_3$).

Example 2: A solution of 21.5 g of isobutyl P-(4-aminobut-2-yl)-P-methyl-phosphinate in 80 ml of 36% aqueous hydrochloric acid is heated to reflux for a period of 10 hours. The reaction mixture is then allowed to cool to room temperature, concentrated under reduced pressure, and co-evaporated twice with 100 ml of water. The crude material is dissolved in water, washed with chloroform, and the aqueous layer treated with

activated charcoal. The aqueous solution is filtered hot, evaporated to dryness, the crude product is dissolved in 50 ml of methanol and treated with 1-2 ml of propylene oxide. The hygroscopic solid is filtered and triturated with acetone. After drying, P-(4-aminobut-2-yl)-P-methyl-phosphinic acid is obtained as a hygroscopic solid, m.p. 68-75°, $^{31}$P-NMR spectrum: $\delta$ = +46.5 ppm ($D_2O$).

The starting material may be prepared as follows:
A solution of 50.0 g of isobutyl P-methylphosphonite and 22.8 g of crotononitrile in 50 ml of dry ethanol is added to a stirred mixture of 0.8 g of sodium hydride (50% dispersion in oil) in 25 ml of ethanol at 0°C, under an atmosphere of nitrogen. The reaction mixture is allowed to warm to room temperature and stirred for 4 hours. 1 ml of glacial acetic acid is added and the mixture is concentrated under reduced pressure. The resulting crude product is dissolved in 50 ml of ethyl acetate, washed twice with 25 ml of water, and the organic extract is dried over magnesium sulphate, and then concentrated under reduced pressure. The crude product is distilled to give isobutyl P-(3-cyanoprop-2-yl)-P-(methyl)-phosphinate, b.p. 110°/0.125 mbar, $^{31}$P-NMR spectrum: $\delta$ = +55.9 and +55.5 ppm ($CDCl_3$).

A solution of 29.8 g of isobutyl P-(3-cyanoprop-2-yl)-P-methyl-phosphinate in 200 ml of ethanol is added to 310.0 g of an 8% solution of ammonia in ethanol. To this are added 20 ml of Raney nickel slurry, and the resulting mixture is hydrogenated at 1 bar until hydrogen uptake ceases. The mixture is then filtered and the filtrate is concentrated under reduced pressure. The crude product is distilled under reduced pressure to give isobutyl P-(4-aminobut-2-yl)-P-methyl-phosphinate, b.p. 100°/0.1 mbar, $^{31}$P-NMR spectrum: $\delta$ = +58.9 and +58.4 ppm ($CDCl_3$).

Example 3: A solution of 9.6 g isobutyl P-(2-hydroxy-3-phthalimido-propyl)-P-methyl-phosphinate in 100 ml of 36% aqueous hydrochloric acid is heated to reflux for a period of 15 hours. The reaction mixture is then allowed to cool to room temperature, concentrated under reduced pressure, and co-evaporated three times with 25 ml of water. The crude material is dissolved in 50 ml of water, washed with 20 ml of ether and the aqueous layer is treated with activated charcoal. The aqueous solution is filtered hot, the filtrate evaporated to dryness and the crude product dissolved in 50 ml of ethanol. 1-2 ml of propylene oxide are added and the solution stirred until the precipitate is free of halogen. Filtration and drying then gives P-(3-amino-2-hydroxy-propyl)-P-(methyl)-phosphinic acid, m.p. 207-208°; $^{31}$P-NMR spectrum: $\delta$ = 38.9 ppm ($D_2O$).

The starting material may be prepared as follows:
To a solution of 12.1 g of isobutyl O-trimethylsilyl-P-methyl-phosphonite in 100 ml of dry tetrahydrofuran are added 11.8g of 2,3-epoxypropylphthalimide followed by 0.5g of dry zinc chloride. The mixture is heated to reflux for a period of 2 hours under an inert gas atmosphere. The mixture is allowed to cool to room temperature, the solvent is evaporated under reduced pressure, the residue dissolved in 100 ml of chloroform, and this is stirred vigorously with 50 ml of water for a period of 0.5 hours. The organic layer is separated, dried over magnesium sulphate and the solvent is removed under reduced pressure. The residue is triturated with 50 ml of hexane:ether 1:1, and the resulting white solid filtered and dried to give isobutyl P-(2-hydroxy-3-phthalimidopropyl)-P-methyl-phosphinate, m.p. 110-113°; $^{31}$P-NMR spectrum: $\delta$ = +54.8 and + 53.5 ppm ($CDCl_3$).

Example 4: A solution of 1.1 g of isobutyl P-[3-amino-2(S)-hydroxy-propyl]-P-methyl-phosphinate in 20 ml of 36% aqueous hydrochloric acid is heated to reflux for a period of 12 hours. The reaction mixture is then allowed to cool to room temperature, concentrated under reduced pressure, and co-evaporated four times with 25 ml of water. The crude material is dissolved in water, washed with ether and the aqueous layer is treated with activated charcoal. The solution is filtered hot, the filtrate is concentrated under reduced pressure, the residue dissolved in 20 ml of ethanol and treated with 1 ml of propylene oxide. The mixture is stirred until the precipitated solid is free of halogen. The solid is then filtered and recrystallised from methanol/acetone give P-[3-amino-2(S)-hydroxy-propyl]-P-methyl-phosphinic acid, m.p. 221-222.5°, $^{31}$P-NMR spectrum: $\delta$ = +38.9 ppm ($D_2O$), $[\alpha]_D^{25}$ = -6.0° (c=0.887% in $H_2O$).

The starting material may be prepared as follows:
To a solution of 4.55 g of isobutyl O-trimethylsilyl-P-methyl-phosphonite in 100 ml of dry tetrahydrofuran are added 5.0g of (2R)-glycidyl tosylate followed by 0.2g of dry zinc chloride. The mixture is heated to reflux for a period of 3 hours under an inert gas atmosphere. The mixture is allowed to cool to room temperature, the solvent is evaporated under reduced pressure, the residue dissolved in 50 ml of chloroform, and this is stirred vigorously with 25 ml of water for a period of 0.5 hours. The organic layer is separated, dried over magnesium sulphate and the solvent is removed under reduced pressure. The residue is chromatographed on silica gel using 5 parts of ethyl acetate and 1 part of ethanol as eluent. There is obtained isobutyl P-[2(S)-hydroxy-3-tosyloxy-propyl]-P-methyl-phosphinate as a viscous oil, $^{31}$P-NMR spectrum: $\delta$ = + 54.5 and + 53.4 ppm ($CDCl_3$), $[\alpha]_D^{25}$ = + 6.5 (c = 0.54% in ethanol).

A solution of 3.32g of isobutyl P-[2(S)-hydroxy-3-tosyloxy-propyl]-P-methyl-phosphinate and 1.19 g of sodium azide in 25 ml of dry dimethylformamide is heated to a temperature of 120° for a period of 3 hours, under an inert gas atmosphere. The reaction mixture is allowed to cool to room temperature, poured onto 50 ml of water and extracted twice with 100 ml of ethyl acetate. The organic extract is dried over magnesium sulphate and the solvent removed under reduced pressure. The residue is chromatographed on silica gel using 5 parts of ethyl acetate to 1 part of ethanol as eluent. There is obtained isobutyl P-[3-azido-2(S)-hydroxy-propyl]-P-methyl-phosphinate as a viscous oil, $^{31}$P-NMR spectrum: $\delta$ = + 54.8 and + 53.7 ppm ($CDCl_3$), $[\alpha]_D^{25}$ = + 18.6 (0.56% in ethanol).

To a solution of 1.2 g of isobutyl P-[3-azido-2(S)-hydroxy-propyl]-P-methyl-phosphinate in 25 ml of ethanol are added 0.25g of 5% palladium on charcoal. The resulting mixture is hydrogenated at 1 bar until hydrogen uptake ceases. The mixture is then filtered and the filtrate evaporated to give isobutyl P-[3-amino-2(S)-hydroxypropyl]-P-(methyl)-phosphinate as a viscous oil, $^{31}$P-NMR spectrum: $\delta$ = + 55.6 and + 54.6 ppm ($CDCl_3$), $[\alpha]_D^{25}$ = + 10.9 (c = 0.50% in ethanol).

Example 5: A solution of 1.6 g of isobutyl P-[3-amino-2(R)-hydroxy-propyl]-P-methyl-phosphinate in 20 ml of 36% aqueous hydrochloric acid is heated to reflux for a period of 12 hours. The reaction mixture is then allowed to cool to room temperature, concentrated under reduced pressure, and co-evaporated four times with 25 ml of water. The crude material is dissolved in water, washed with ether and the aqueous layer is treated with activated charcoal. The solution is filtered hot, the filtrate is concentrated under reduced pressure, the residue dissolved in 20 ml of ethanol and treated with 1 ml of propylene oxide. The mixture is stirred until the precipitated solid is free of halogen. The solid is then recrystallised from methanol/acetone to give P-[3-amino-2(R)-hydroxy-propyl]-P-methyl-phosphinic acid, m.p. 222-225°, $^{31}$P-NMR spectrum: $\delta$ = + 38.8 ppm ($D_2O$), $[\alpha]_D^{25}$ = + 5.9 (c = 0.918% in $H_2O$).

The starting material may be prepared as follows:

To a solution of 4.55 g of isobutyl O-trimethylsilyl-P-methyl-phosphonite in 100 ml of dry tetrahydrofuran are added 5.0g of (2S)-glycidyl tosylate followed by 0.2g of dry zinc chloride. The mixture is heated to reflux for a period of 3 hours under an inert gas atmosphere. The mixture is allowed to cool to room temperature, the solvent is evaporated under reduced pressure, the residue dissolved in 50 ml of chloroform, and this is stirred vigorously with 25 ml of water for a period of 0.5 hours. The organic layer is separated, dried over magnesium sulphate and the solvent is removed under reduced pressure. The residue is chromatographed on silica gel using 5 parts of ethyl acetate and 1 part of ethanol as eluent. There is obtained isobutyl P-[2(R)-hydroxy-3-tosyloxy-propyl]-P-methyl-phosphinate as a viscous oil, $^{31}$P-NMR spectrum: $\delta$ = 54.5 and + 53.4 ppm ($CDCl_3$), $[\alpha]_D^{25}$ = - 6.8 (c = 0.44% in ethanol).

A solution of 4.3g of isobutyl P-[2(R)-hydroxy-3-tosyloxy-propyl]-P-methyl-phosphinate and 1.5 g of sodium azide in 25 ml of dry dimethylformamide is heated to a temperature of 120° for a period of 3 hours under an inert gas atmosphere. The reaction mixture is allowed to cool to room temperature, poured onto 50 ml of water and extracted twice with 100 ml of ethyl acetate. The organic extract is dried over magnesium sulphate and the solvent removed under reduced pressure. The residue is chromatographed on silica gel using 5 parts of ethyl acetate to 1 part of ethanol as eluent. There is obtained isobutyl P-[3-azido-2(R)-hydroxy-propyl]-P-methyl-phosphinate as a viscous oil, $^{31}$P-NMR spectrum: $\delta$ = +54.8 and + 53.7 ppm ($CDCl_3$), $[\alpha]_D^{25}$ = - 15.8 (0.51% in ethanol).

To a solution of 2.2 g of isobutyl P-[3-azido-2(R)-hydroxy-propyl]-P-methyl-phosphinate in 25 ml of ethanol is added 0.25g of 5% palladium on charcoal. The resulting mixture is hydrogenated at 1 bar until hydrogen uptake ceases. The mixture is then filtered and the filtrate evaporated to give isobutyl P-[3-amino-2(R)-hydroxy-propyl]-P-methyl-phosphinate as a viscous oil, $^{31}$P-NMR spectrum: $\delta$ = + 55.6 and + 54.6 ppm ($CDCl_3$), $[\alpha]_D^{25}$ = -9.9 (0.66% in ethanol).

Example 6: A solution of 0.5g of isobutyl P-(3-tert.-butoxycarbonylamino-2-oxo-propyl)-P-methyl-phosphinate in 10 ml of 36% aqueous hydrochloric acid is heated to reflux for a period of 4 hours. The mixture is then allowed to cool to room temperature, concentrated under reduced pressure, and co-evaporated twice with 20 ml of water. The crude product is dissolved in 20 ml of ethanol, 1 ml of propylene oxide is added, and the mixture is stirred until the precipitated solid is free of halogen. The solid is filtered and dried to give P-(3-amino-2-oxo-propyl)-P-methyl-phosphinic acid, m.p. 148-149°, $^{31}$P-NMR spectrum: $\delta$ = 32.8 ppm ($D_2O$).

The starting material may be prepared as follows:

To a solution of 6.1g of diisopropylamine in 25 ml of dry tetrahydrofuran at 0° under an atmosphere of nitrogen are added 37.5 ml of a 1.6M solution of n-butyllithium in hexane. This solution is stirred for a period of 10 minutes and then cooled to -78°. To this is added, via a syringe, a solution of 9.0g of isobutyl P,P-dimethyl-phosphinate in 50 ml of dry tetrahydrofuran and the mixture is stirred at -78° for a period of 1 hour. To this is then added a solution of 1.9g of methyl N-tert.-butoxycarbonylaminoglycinate in 25 ml of

dry tetrahydrofuran and the reaction mixture is allowed to warm to room temperature and is stirred for a period of 1 hour. 3 ml of glacial acetic acid are then introduced, followed by 50 ml of saturated sodium bicarbonate solution, and the aqueous layer is extracted twice with 100 ml of ether. The organic extract is dried over magnesium sulphate and concentrated under reduced pressure. The residue is chromatographed on silica gel using ethyl acetate as eluent. There is obtained isobutyl P-(3-t-butoxycarbonylamino-2-oxo-propyl)-P-(methyl)-phosphinate, m.p. 65-68°, $^{31}$P-NMR spectrum: $\delta$ = + 44.9 ppm ($CDCl_3$).

Example 7: A solution of 4.0g of ethyl P-(3-benzyloxycarbonylamino-1-hydroxy-propyl)-P-methyl-phosphinate in 50 ml of 5.0M aqueous hydrochloric acid is heated to reflux for 20 hours under an inert gas atmosphere. Then, the reaction mixture is cooled to room temperature and washed twice with 100ml each of dichloromethane and once with diethyl ether. The aqueous layer is evaporated to dryness at 50° under reduced pressure. The oily residue is then co-evaporated 5 times with 50 ml each of water and of absolute ethanol. The remaining white solid is dried under reduced pressure at 80° and then re-crystallised to afford P-(3-amino-1-hydroxy-propyl)-P-methyl-phosphinic acid hydrochloride of m.p. 115-116.5°. This can be converted into the free compound by dissolving in ethanol and treating with propylene oxide yielding, after filtration and drying, P-(3-amino-1-hydroxy-propyl)-P-methyl-phosphinic acid of m.p. 125-126.5°.

The starting material may be prepared as follows:

A mixture of 5.18g of 3-(benzyloxycarbonylamino)propionaldehyde, 2.7g of ethyl P-methylphosphinate and 2,53g of triethylamine is heated to 100° under an inert gas atmosphere for 2 hours. After cooling to room temperature the volatile materials are removed under reduced pressure to afford a viscous oil. Chromatography thereof on silica gel gives ethyl P-(3-benzyloxycarbonylamino-1-hydroxy-propyl)-P-methyl-phosphinate as a colourless, viscous oil.

Example 8: A mixture of 520 mg (2.6 mmol) of ethyl P-(3-aminopropyl)-P-difluoromethyl-phosphinate and 5 ml of 12M hydrochloric acid is refluxed for 3 hours and then evaporated to dryness. The residue is dissolved in 5 ml of methanol. To the stirred solution 25 ml of epoxypropane are added dropwise, upon which spontaneous crystallisation occurs. The crystals are collected and dried yielding P-(3-aminopropyl)-P-difluoromethyl-phosphinic acid of m.p. 261°.

The starting material can be prepared as follows:

To a suspension of 15.8 g of sodium hydride in 500 ml of dry tetrahydrofuran 67 g (300 mmol) of ethyl P-(1,1-diethoxyethyl)phosphinate are added dropwise at such a rate that the reaction temperature does not exceed 25°. The reaction mixture is stirred for 1 hour at room temperature and cooled to -10°. Then 77.8 g (900 mmol) of chlorodifluoromethane are added. Stirring is continued for additional 2 hours, upon which 100 ml of ice-cold water are added. The reaction mixture is extracted 3-times with 500 ml each of dichloromethane. The extracts are combined, dried over magnesium sulphate, filtrated and evaporated to dryness yielding ethyl P-(2,2-diethoxyethyl)-P-difluoromethyl-phosphinate as a viscous oil of $R_F$ = 0.44 (dichloromethane/ethyl acetate; 9:1).

A mixture of 5 ml of dry ethanol and 10.9 ml (86.5 mmol) of trimethylchlorosilane is added to a solution of 15 g (57.6 mmol) of ethyl P-(2,2-diethoxyethyl)-P-difluoromethylphosphinate in 95 ml of dry dichloromethane. The reaction mixture is stirred for 3 hours at room temperature and then evaporated to dryness yielding ethyl P-difluoromethylphosphinate of $R_f$ = 0.1 (ethyl acetate).

660 mg (28.8 mmol) of sodium are dissolved in 40 ml of ethanol. The solution is cooled to -10° and 8.6 g (57.6 mmol) of ethyl P-difluoromethylphosphinate and 3.8 ml (57.6 mmol) of acetonitrile are added with stirring. The reaction mixture is then allowed to warm up to room temperature and stirred for additional 17 hours and then adjusted to pH 6 by addition of glacial acetic acid. The solvents are evaporated and the residue is dissolved in dichloromethane, washed twice with water, dried over magnesium sulphate and evaporated to dryness. The crude product is purified by chromatography on silica gel with ethyl acetate/dichloromethane (7:3) as eluent. The fractions containing the desired product are combined and evaporated to dryness yielding ethyl P-(2-cyanoethyl)-P-difluoromethylphosphinate as an oil of $R_f$ = 0.54 (above eluent).

A solution of 1.0 g (5.1 mmol) of ethyl P-(2-cyanoethyl)-P-difluoromethyl-phosphinate in 10 ml of dry ethanol is treated with 4 g of liquid ammonia and 0.3 g of Raney nickel. The reaction mixture is hydrogenated at 50° for 9 hours at 100 mbar. The reaction mixture is cooled, filtrated and evaporated to dryness. Chromatographic purification yields ethyl P-(3-aminopropyl)-P-difluoromethyl-phosphinate as a colourless oil of $R_f$ = 0.22 (dichloromethane/methanol/aqueous ammonia; 80:19:1).

Example 9: A mixture of 4,53 g (30 mmol) of P-(5-aminopent-3-yl)phosphonous acid and 24.21 g (150 mmol) of hexamethyldisilazane is refluxed under argon while stirring for 16 hours. To the resulting solution 15 ml of diethylene glycol dimethyl ether are added and boiling is continued for additional 2 hours. The reaction mixture is cooled to 100° and 19.38 g (150 mmol) of N-ethyl-N,N-diisopropyl-amine are added over a period of 20 minutes. After cooling to 25°, 21.29g (15 mmol) of methyliodide are added over a

period of 20 minutes, the reaction temperature being kept at 25° with external cooling. The reaction mixture is stirred for 4 days, then cooled to 10°. The white precipitate is filtered off. The filtrate is evaporated under reduced pressure, and the residue diluted with 100 ml of cold dichloromethane and extracted three times with 50 ml each of 2N hydrochloric acid. The extracts are combined, evaporated to dryness and co-evaporated 2 additional times with 50 ml each of water to give a colourless oil. This oil is dissolved in 50 ml of methanol, 300 ml of propylene oxide are added and the mixture is kept at 4° overnight and then evaporated under reduced pressure. The crude product is purified by chromatography on 150g of Opti-Up® $C_{12}$ with water as eluent. The fractions containing the desired product are combined and evaporated under reduced pressure. The solid residue is dried under reduced pressure yielding P-(5-aminopent-3-yl)-P-methylphosphinic acid x 0.52 $H_2O$ (hygroscopic).

The starting material can be prepared in the following manner:

2.90 g (0.126 mol) of sodium are dissolved in 72 ml of ethanol. At from 0 to +5° 58.6 g (0.3 mol) of ethyl P-(diethoxymethyl)phosphonite and 42.3 ml (0.3 mol) of pent-2-enenitrile, dissolved in 72 ml of ethanol, are added while stirring over a period of 6 hours. The mixture is then allowed to warm to room temperature and stirring is continued for 16 hours. 7 ml of glacial acetic acid are added at 10°. Then, the solvent is removed under reduced pressure. The residue is dissolved in ethyl acetate, washed twice with water and dried over sodium sulphate. Evaporation in vacuo yields the cruder product as a yellow oil. After distillation at 100°/0.01 Torr ethyl P-(4-cyano(but-3-yl)-P-(diethoxymethyl)-phosphinate is obtained as a colourless oil.

73.4 g (0.264 mol) of ethyl P-(4-cyanobut-3-yl)-P-(diethoxymethyl)-phosphinate in 770 ml of dry ethanol are treated with 126 g of an 8% solution of ammonia in ethanol. Subsequently, 15 g of Raney-nickel and the resulting mixture is hydrogenated at 45° under atmospheric pressure. The catalyst is then filtered of and the filtrate is concentrated under reduced pressure. The crude product is distilled in vacuo to yield the ethyl P-(5-aminopent-3-yl)-P-(diethoxymethyl)-phosphinate (b.p.: 100°/0.01 Torr).

A solution of 61.88 g (0.22 mol) of ethyl P-(5-aminopent-3-yl)-P-(diethoxymethyl)-phosphinate in 220 ml of 36% aqueous hydrochloric acid is heated to reflux for a period of 6 hours. The reaction mixture is then allowed to cool to room temperature, concentrated under reduced pressure, and co-evaporated three times with 10 ml-portions of water. The crude material is dissolved in 100 ml of methanol, and 500 ml of propylene oxide are added while stirring. The mixture is left to stand overnight at 4° and the white precipitate is then filtered off and recrystallized from methanol/acetone to give pure hygroscopic P-(5-aminopent-3-yl)phosphorous acid [m.p.: 130-140° (decomposition)].

Example 10: To a mixture of 825 mg of diisopropylaminomethyl-polystyrene in 5 ml of acetonitrile are added 69.4 mg (0.2 mmol) of ethyl P-(4-amino-1,1,1-trifluoro-but-2-yl)-P-methyl-phosphinate trifluoroacetate while stirring at 25°. To this mixture are added 0.09 ml (0.7 mmol) of trimethylsilyl bromide. After stirring for 1 hour at 25°, the mixture is filtered and 299.78 mg (2 mmol) of sodium iodide and 217.28 mg (2 mmol) of trimethylsilyl chloride are added to the filtrate which is then stirred for 16 hours at 25°. The sodium chloride precipitated is filtered off and the filtrate is evaporated under reduced pressure to dryness. The crude product is dissolved in 2 ml of acetonitrile. 15 mg (0.83 mmol) of water are added to the resulting solution. After stirring for 1 hour at 25°, the solution is evaporated under reduced pressure to dryness and chromatographed on 50 g of Opti-Up® $C_{12}$ with acetonitrile as eluent to remove a small amount of the starting material. After re-eluting with water, the product-containing fractions are combined and evaporated under reduced pressure to give P-(4-amino-1,1,1-trifluoro-but-2-yl)-P-methyl-phosphinic acid hydroiodide as an oil; $^1$H-NMR spectrum: $\delta$ = 3.20 ppm (m, 2H), 2.67 ppm (m, 1H), 2.15 (m, 2H), 1.42 ppm (d, 3H).

The starting material can be obtained, for example, as follows:

A solution of 2.16 g (20 mmol) of O-ethyl-P-methyl-phosphonous acid and 4.05 g (40 mmol) of triethylamine in 100 ml of dry tetrahydrofuran is stirred under an atmosphere of argon at 25°. To this solution are added at 25° 4.35 g (40 mmol) of trimethylsilyl chloride over a period of 10 minutes. A white precipitate is formed. The reaction mixture is stirred at 25° for additional 16 hours. Then, 2.42 g (20 mmol) of 4,4,4-trifluorocrotononitrile, dissolved in 20 ml of dry tetrahydrofuran, are added at 25° over a period of 10 minutes. The reaction mixture is refluxed for 40 hours, cooled to 25°, poured into ice-water and extracted with dichloromethane. The extracts are combined, washed with water, dried over anhydrous sodium sulphate and evaporated under reduced pressure to dryness. The crude product is chromatographed on 200 g of silica gel with trichloromethane as eluent. The product-containing fractions are combined and evaporated to give ethyl P-(3-cyano-1,1,1-trifluoro-prop-2-yl)-P-methyl-phosphinate as an oil.

250 mg of platinum oxide are added to a solution of 458.28 mg (2 mmol) of ethyl P-(3-cyano-1,1,1-trifluoro-prop-2-yl)-P-methyl-phosphinate dissolved in 28 ml of trifluoroacetic acid, and the resulting mixture is hydrogenated at 25° and 4 bar. The catalyst is filtered off and the filtrate is evaporated to dryness under reduced pressure. The resulting crude product is chromatographed on 70 g of Opti-Up® $C_{12}$ with acetonitrile as the eluent. The product-containing fractions are combined and evaporated under reduced

pressure to give ethyl P-(4-amino-1,1,1-trifluoro-but-2-yl)-P-methyl-phosphinate trifluoroacetate as an oil.

Example 11: The hydrogenation of P-(3-aminopropen-1-yl)-P-methyl-phosphinic acid carried out by conventional hydrogenation techniques well known in the art yields P-(3-aminopropyl)-P-methyl-phosphinic acid, identical with the product obtained in Example 1.

The starting material can be prepared, for example, as follows:

A solution of 2.7 g of diisobutyl P,P-(dimethyl)-methylenebisphosphinate in 25 ml of dry tetrahydrofuran is added to a suspension of 0.23 g of sodium hydride in 10 ml of dry tetrahydrofuran under an inert gas atmosphere. The mixture is stirred at room temperature under an inert gas atmosphere until the gas evolution ceases. The mixture is then added to a solution of 1.8 g of N-(formylmethyl)-phthalimide in 25 ml of dry tetrahydrofuran at 0° under an inert gas atmosphere. The reaction mixture is allowed to warm to room temperature and then stirred for one hour. 5 ml of saturated ammonium chloride solution are added. The mixture is extracted twice with 25 ml-portions of diethyl ether. The combined organic extracts are dried over magnesium sulphate. The solvent is removed under reduced pressure and the resulting residue purified by chromatography on silica gel using 5 parts of ethyl acetate to 1 part of ethanol as eluent. The fractions containing product are combined and concentrated under reduced pressure to give isobutyl P-(3-phthalimidopropen-1-yl)-P-methyl-phosphinate as a viscous oil [$^{31}$P-NMR spectrum: $\delta$ = +34.1 ppm ($CDCl_3$)].

A solution of 0.72 g of isobutyl P-(3-phthalimidopropen-1-yl)-P-methyl-phosphinate in 25 ml of aqueous hydrochloric acid (36%) is heated to reflux for 15 hours. The reaction mixture is then allowed to cool to room temperature. Some insoluble material is removed by filtration and the filtrate is concentrated under reduced pressure. The resulting crude material is co-evaporated four times with 25 ml-portions of water, dissolved in 25 ml of ethanol and treated with 1-2 ml of propylene oxide. The precipitated solid is removed by filtration and purified by chromatography on Dowex 50W X2 resin with water as eluent. The fractions containing the desired product are combined and evaporated and the resulting solid is dried to give P-(3-aminopropen-1-yl)-P-methyl-phosphinic acid of m.p. 209-213° [$^{31}$P-NMR spectrum: $\delta$ = +30.4 ppm ($D_2O$)].

Example 12: In an analogous manner as described in any one of Examples 1 to 11, also the following compounds can be manufactured:

P-(3-aminopropyl)-P-fluoromethyl-phosphinic acid and

P-(3-aminopropyl)-P-trifluoromethyl-phosphinic acid.

Example 13: Tablets, each containing 75 mg of the active ingredient, for example, P-(3-amino-2-oxo-propyl)-P-methyl-phosphinic acid, can be manufactured in the following manner:

| Constituents (for 1000 tablets) | |
|---|---|
| active ingredient 75.0 g lactose | 268.5 g |
| corn starch | 22.5 g |
| polyethylene glycol 6000 | 5.0 g |
| talcum | 15.0 g |
| magnesium stearate | 4.0 g |
| demineralised water | q.s. |

Preparation: The solid ingredients are first forced through a sieve of 0.6 mm mesh width. Then, the active ingredient, lactose, talcum, magnesium stearate and half of the starch are homogeneously mixed. The other half of the starch is suspended in 65 ml of water, and this suspension is added to a boiling solution of the polyethylene glycol in 260 ml of water. The resulting paste is added to the pulverulent substances, and the whole is mixed and granulated, if necessary with the addition of water. The granulate is dried overnight at 35°, forced through a sieve of 1.2 mm mesh width and compressed into tablets of approximately 10 mm diameter which are concave on both sides and have a breaking notch on the upper side.

Example 14: Tablets, each containing 10 mg of the active ingredient, for example, P-(3-amino-2-oxo-propyl)-P-(methyl)-phosphinic acid, can be prepared in the following manner:

| Constituents (for 1000 tablets) | |
|---|---|
| active ingredient | 10.0 g |
| lactose | 25.0 g |
| corn starch | 308.5 g |
| polyethylene glycol 6000 | 32.5 g |
| talcum | 10.0 g |
| magnesium stearate | 15.0 g |
| demineralised water | q.s. |

Preparation: The solid ingredients are first forced through a sieve of 0.6 mm mesh width. Then, the active ingredient, lactose, talcum, magnesium stearate and half of the starch are homogeneously mixed. The other half of the starch is suspended in 65 ml of water, and this suspension is added to a boiling solution of the polyethylene glycol in 260 ml of water. The resulting paste is added to the pulverulent substances, and the whole is mixed and granulated, if necessary with the addition of water. The granulate is dried overnight at 35°, forced through a sieve of 1.2 mm mesh width and compressed into tablets of approximately 10 mm diameter which are concave on both sides and have a breaking notch on the upper side.

Example 15: Gelatine dry-filled capsules, each containing 150 mg of the active ingredient, for example, P-(3-amino-2-oxo-propyl)-P-methyl-phosphinic acid, can be prepared in the following manner:

| Constituents (for 1000 capsules) | |
|---|---|
| active ingredient | 150.0 g |
| microcrystalline cellulose | 30.0 g |
| sodium lauryl sulphate | 2.0 g |
| magnesium stearate | 8.0 g |

The sodium lauryl sulphate is added to the active ingredient (lyophilised) through a sieve of mesh width 0.2 mm and these two components are intimately mixed for 10 minutes. The microcrystalline cellulose is then added through a sieve of mesh width 0.9 mm and the mixture is again intimately mixed for 10 minutes. Finally, the magnesium stearate is added through a sieve of mesh width 0.8 mm and, after mixing for additional 3 minutes, the mixture is introduced into size 0 (elongated) gelatine dry-filled capsules in portions of 390 mm.

Example 16: A 0.2% injection or infusion solution of the active ingredient, for example, of P-(3-amino-2-oxo-propyl)-P-methyl-phosphinic acid, can be prepared in the following manner:

| Constituents (for 2500 ml) | |
|---|---|
| active ingredient | 5.0 g |
| sodium chloride | 22.5 g |
| phosphate buffer pH = 7.4 | 300.0 g |
| demineralised water | ad 2500.0 ml |

The active ingredient and the sodium chloride are dissolved in 1000 ml of water and filtered through a microfilter. The buffer solution is added and then water is added to give a volume of 2500 ml. For the preparation of dosis unit forms, portions of 1.0 or 2.5 ml are introduced into glass ampoules (each containing 2.0 or 5.0 mg of the active ingredient, respectively).

## Claims

**Claims for the following Contracting States : AT, BE, CH, DE, DK, FR, GB, IT, LI, LU, NL, SE**

**1.** A P-substituted aminoalkylphosphinic acid of the formula

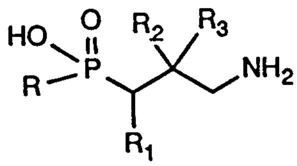

(I),

or a salt thereof, wherein R denotes an optionally fluorinated methyl group, $R_1$ denotes hydrogen, lower alkyl, lower alkoxy, hydroxy, halogen or a fluorinated methyl group and $R_2$ and $R_3$ denote hydrogen or $R_2$ denotes hydroxy, lower alkoxy or halogen and $R_3$ is hydrogen or $R_2$ and $R_3$ together represent an oxo group, with the exception of P-(3-amino-2-oxo-propyl)-P-methyl-phosphinic acid, racemic P-(3-amino-2-hydroxypropyl)-P-methyl-phosphinic acid, P-(3-aminopropyl)-P-methyl-phosphinic acid and acid addition salts, alkali metal salts, the magnesium salt and the ammonium salt of P-(3-aminopropyl)-P-methyl-phosphinic acid.

**2.** A compound according to claim 1, wherein R denotes methyl, fluoromethyl, difluoromethyl or trifluoromethyl, $R_1$ denotes hydrogen, lower alkyl, lower alkoxy, hydroxy, halogen, fluoromethyl, difluoromethyl or trifluoromethyl and $R_2$ and $R_3$ denote hydrogen or $R_2$ denotes hydroxy, lower alkoxy or halogen and $R_3$ denotes hydrogen or $R_2$ and $R_3$ together represent oxo.

**3.** A compound according to claim 1, wherein R denotes methyl, fluoromethyl, difluoromethyl, $R_1$ denotes hydrogen or $C_1$-$C_4$alkyl, $R_2$ denotes hydrogen or hydroxy and $R_3$ denotes hydrogen or $R_2$ and $R_3$ together represent oxo.

**4.** A compound according to claim 1, wherein R denotes methyl, $R_1$ is hydrogen, and wherein $R_2$ represents hydrogen or hydroxy and $R_3$ is hydrogen or $R_2$ and $R_3$ together represent oxo, provided that, when $R_2$ denotes hydroxy, the C-atom to which it is attached has S-configuration, in the free form or in the form of an acid addition salt.

**5.** A compound of the formula I, wherein R denotes methyl, fluoromethyl, difluoromethyl or trifluoromethyl, $R_1$ denotes hydroxy and $R_2$ and $R_3$ are hydrogen, or a salt thereof.

**6.** P-[3-amino-2(R)-hydroxy-propyl]-P-methyl-phosphinic acid or an acid addition or base salt thereof.

**7.** P-[3-amino-2(S)-hydroxy-propyl]-P-methyl-phosphinic acid or an acid addition or base salt thereof.

**8.** P-(5 aminopent-3-yl)-P-methyl-phosphinic acid or an acid addition or base salt thereof.

**9.** P-(4-amino-1,1,1-trifluoro-but-2-yl)-P-methyl-phosphinic acid or an acid addition or base salt thereof.

**10.** P-(3-aminopropyl)-P-fluoromethyl-phosphinic acid or an acid addition or base salt thereof.

**11.** P-(3-aminopropyl)-P-difluoromethyl-phosphinic acid or an acid addition or base salt thereof.

**12.** P-(3-aminopropyl)-P-trifluoromethyl-phosphinic acid or an acid addition or base salt thereof.

**13.** An acid addition or base salt of P-(3-amino-2-oxo-propyl)-P-methyl-phosphinic acid.

**14.** P-(4-aminobut-2-yl)-P-methyl-phosphinic acid or an acid addition or base salt thereof.

**15.** P-(3-amino-1-hydroxy-propyl)-P-methyl-phosphinic acid or an acid addition or base salt thereof.

**16.** A compound for use in a method for the treatment of the human or animal body, said compound being a P-substituted aminoalkylphosphinic acid of the formula

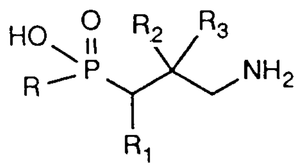

(I),

or a pharmaceutically acceptable salt thereof, with the exception of P-(3-aminopropyl)-P-methyl-phosphinic acid and acid addition salts, alkali metal salts and the magnesium salt thereof, wherein R denotes an optionally fluorinated methyl group, $R_1$ denotes hydrogen, lower alkyl, lower alkoxy, hydroxy, halogen or a fluorinated methyl group and $R_2$ and $R_3$ denote hydrogen or $R_2$ denotes hydroxy, lower alkoxy or halogen and $R_3$ is hydrogen or $R_2$ and $R_3$ together represent an oxo group.

**17.** A compound for use according to claim 16, said compound being P-(3-aminopropyl)-P-methyl-phosphinic acid in the form of a pharmaceutically acceptable ammonium salt thereof.

**18.** A compound for use according to claim 16, said compound being P-(3-amino-2-hydroxy-propyl)-P-methyl-phosphinic acid in the free form or in the form of a pharmaceutically acceptable salt thereof.

**19.** A compound for use according to claim 16, said compound being P-(3-amino-2-oxo-propyl)-P-methyl-phosphinic acid in the free form or in the form of a pharmaceutically acceptable salt thereof.

**20.** A compound according to any one of claims 2 to 15 for use according to claim 16.

**21.** A pharmaceutical composition containing a compound according to any one of claims 1 to 20 in admixture with conventional pharmaceutical carriers.

**22.** A process for the manufacture of compounds according to claim 1, characterised in:
a) in a compound of formula

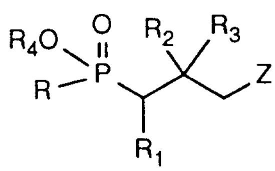

(II),

in which R, $R_1$, $R_2$ and $R_3$ have their previous significances, Z is $-NH_2$ and $R_4$ is a hydroxy-protective group $R_5$ or, when R is methyl and $R_1$, $R_2$ and $R_3$ are hydrogen, $R_4$ is an alkali metal or ammonium ion $R_6$, or Z is a protected or latent amino group $Z_0$, and $R_4$ is hydrogen or a hydroxy-protective group $R_5$, and wherein a carbonyl group formed by $R_2$ and $R_3$ together with the carbon atom to which they are attached may also be present in a temporarily protected form, any group $R_5$ or $R_6$ is replaced by hydrogen and/or any group $Z_0$ is converted into $-NH_2$ and/or, if $R_2$ and $R_3$ together with the carbon atom to which they are attached form a protected carbonyl group, such protecting group is removed; or
b) in a compound of the formula

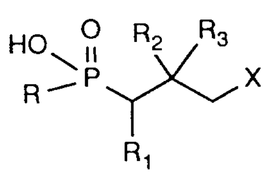

(III),

in which R, $R_1$, $R_2$ and $R_3$ have their previous significances and X is a group capable of being converted into the group of formula $-CH_2-NH_2$(Ia), the group X is converted into the group (Ia); or
c) a compound of formula I' being identical to a corresponding compound of formula I apart from having one or more carbon-carbon multiple bond(s) is reduced to produce a compound of formula I wherein R has its previous significance, $R_1$ is hydrogen, lower alkyl or fluorinated methyl and $R_2$ and $R_3$ are hydrogen, and, if desired, a resulting compound is converted into another compound of the formula I, a resulting mixture of isomers is separated into the individual isomers and/or a resulting salt obtained in this process is converted into the free compound of the formula I or into another salt and/or, if desired, a resulting free compound of the formula I is converted into a salt to correspond to the above definition.

**Claims for the following Contracting States : ES, GR**

**1.** A process for the manufacture of a compound of formula (I)

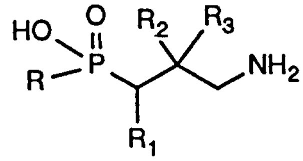

(I),

or a salt thereof, wherein R denotes an optionally fluorinated methyl group, $R_1$ denotes hydrogen, lower alkyl, lower alkoxy, hydroxy, halogen or a fluorinated methyl group and $R_2$ and $R_3$ denote hydrogen or $R_2$ denotes hydroxy, lower alkoxy or halogen and $R_3$ is hydrogen or $R_2$ and $R_3$ together represent an oxo group, with the exception of P-(3-amino-2-oxo-propyl)-P-methyl-phosphinic acid, racemic P-(3-amino-2-hydroxy-propyl)-P-methyl-phosphinic acid, P-(3-aminopropyl)-P-methyl-phosphinic acid and acid addition salts, alkali metal salts, the magnesium salt and the ammonium salt of P-(3-aminopropyl)-P-methyl-phosphinic acid, characterised in that
a) in a compound of formula

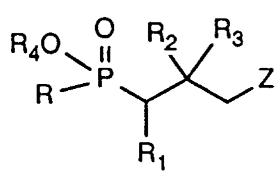

(II),

in which R, $R_1$, $R_2$ and $R_3$ have their previous significances, Z is $-NH_2$ and $R_4$ is a hydroxy-protective group $R_5$ or, when R is methyl and $R_1$, $R_2$ and $R_3$ are hydrogen, $R_4$ is an alkali metal or ammonium ion $R_6$, or Z is a protected or latent amino group $Z_0$, and $R_4$ is hydrogen or a hydroxy-protective group $R_5$, and wherein a carbonyl group formed by $R_2$ and $R_3$ together with the carbon atom to which they are attached may also be present in a temporarily protected form, any group $R_5$ or $R_6$ is replaced by hydrogen and/or any group $Z_0$ is converted into $-NH_2$ and/or, if $R_2$ and $R_3$ together with the carbon atom to which they are attached form a protected carbonyl group, such protecting group is removed; or
b) in a compound of the formula

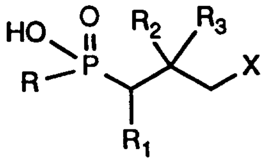

(III),

in which R, $R_1$, $R_2$ and $R_3$ have their previous significances and X is a group capable of being converted into the group of formula $-CH_2-NH_2$(Ia), the group X is converted into the group (Ia); or
c) a compound of formula I' being identical to a corresponding compound of formula I apart from having one or more carbon-carbon multiple bond(s) is reduced to produce a compound of formula I wherein R has its previous significance, $R_1$ is hydrogen, lower alkyl or fluorinated methyl and $R_2$ and $R_3$ are hydrogen, and, if desired, a resulting compound is converted into another compound of the formula I, a resulting mixture of isomers is separated into the individual isomers and/or a resulting salt obtained in this process is converted into the free compound of the formula I or into another salt and/or, if desired, a resulting free compound of the formula I is converted into a salt to correspond to the above definition.

**2.** A process according to claim 1 for the manufacture of a compound of formula (I), wherein R denotes methyl, fluoromethyl, difluoromethyl or trifluoromethyl, $R_1$ denotes hydrogen, lower alkyl, lower alkoxy, hydroxy, halogen, fluoromethyl, difluoromethyl or trifluoromethyl and $R_2$ and $R_3$ denote hydrogen or $R_2$ denotes hydroxy, lower alkoxy or halogen and $R_3$ denotes hydrogen or $R_2$ and $R_3$ together represent oxo.

**3.** A process according to claim 1 for the manufacture of a compound of formula (I) wherein R denotes methyl, fluoromethyl, difluoromethyl or trifluoromethyl, $R_1$ denotes hydrogen or $C_1$-$C_4$alkyl, $R_2$ denotes hydrogen or hydroxy and $R_3$ denotes hydrogen or $R_2$ and $R_3$ together represent oxo.

**4.** A process according to claim 1 for the manufacture of a compound of formula (I) wherein R denotes methyl, $R_1$ is hydrogen, and wherein $R_2$ represents hydrogen or hydroxy and $R_3$ is hydrogen or $R_2$ and $R_3$ together represent oxo, provided that, when $R_2$ denotes hydroxy, the C-atom to which it is attached has S-configuration, in the free form or in the form of an acid addition salt.

**5.** A process according to claim 1 for the manufacture of a compound of the formula I wherein R denotes methyl, fluoromethyl, difluoromethyl or trifluoromethyl, $R_1$ denotes hydroxy and $R_2$ and $R_3$ are hydrogen, or a salt thereof.

**6.** A process according to claim 1 for the manufacture of P-[3-amino-2(R)-hydroxy-propyl]-P-methyl-phosphinic acid or an acid addition or base salt thereof.

**7.** A process according to claim 1 for the manufacture of P-[3-amino-2(S)-hydroxy-propyl]-P-methyl-phosphinic acid or an acid addition or base salt thereof.

**8.** A process according to claim 1 for the manufacture of P-(5 aminopent-3-yl)-P-methyl-phosphinic acid or an acid addition or base salt thereof.

**9.** A process according to claim 1 for the manufacture of P-(4-amino-1,1,1-trifluoro-but-2-yl)-P-methyl-phosphinic acid or an acid addition or base salt thereof.

**10.** A process according to claim 1 for the manufacture of P-(3-aminopropyl)-P-fluoromethyl-phosphinic acid or an acid addition or base salt thereof.

**11.** A process according to claim 1 for the manufacture of P-(3-aminopropyl)-P-difluoromethyl-phosphinic acid or an acid addition or base salt thereof.

**12.** A process according to claim 1 for the manufacture of P-(3-aminopropyl)-P-trifluoromethyl-phosphinic acid or an acid addition or base salt thereof.

**13.** A process according to claim 1 for the manufacture of an acid addition or base salt of P-(3-amino-2-oxo-propyl)-P-methyl-phosphinic acid.

**14.** A process according to claim 1 for the manufacture of P-(4-aminobut-2-yl)-P-methyl-phosphinic acid or an acid addition or base salt thereof.

**15.** A process according to claim 1 for the manufacture of P-(3-amino-1-hydroxy-propyl)-P-methyl-phosphinic acid or an acid addition or base salt thereof.

**16.** A process for the manufacture of a pharmaceutical composition, wherein a compound obtainable according to any of claims 1 to 15 is admixed with a conventional pharmaceutical carrier.

**17.** A process for the manufacture of a pharmaceutical composition, wherein P-(3-aminopropyl)-P-methyl-phosphinic acid in the form of a pharmaceutically acceptable ammonium salt thereof is admixed with a conventional pharmaceutical carrier.

**18.** A process for the manufacture of a pharmaceutical composition, wherein P-(3-amino-2-hydroxy-propyl)-P-methyl-phosphinic acid in the free form or in the form of a pharmaceutically acceptable salt thereof is admixed with a conventional pharmaceutical carrier.

**19.** A process for the manufacture of a pharmaceutical composition, wherein P-(3-amino-2-oxo-propyl)-P-methyl-phosphinic acid in the free form or in the form of a pharmaceutically acceptable salt thereof is admixed with a conventional pharmaceutical carrier.

**Patentansprüche**

**Patentansprüche für folgende Vertragsstaaten : AT, BE, CH, DE, DK, FR, GB, IT, LI, LU, NL, SE**

**1.** P-substituierte Aminoalkylphosphinsäure der Formel

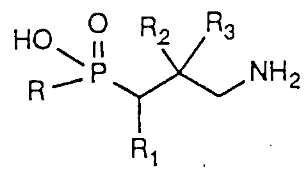

(I),

oder ein Salz hiervon, worin R für eine gegebenenfalls fluorierte Methylgruppe steht, $R_1$ Wasserstoff, Niedrigalkyl, Niedrigalkoxy, Hydroxy, Halogen oder eine fluorierte Methylgruppe bedeutet und $R_2$ und $R_3$ für Wasserstoff stehen oder $R_2$ Hydroxy, Niedrigalkoxy oder Halogen bedeutet und $R_3$ für Wasserstoff steht oder $R_2$ und $R_3$ gemeinsam eine Oxogruppe darstellen mit Ausnahme von P-(3-Amino-2-oxo-propyl)-P-methyl-phosphinsäure, racemischer P-(3-Amino-2-hydroxy-propyl)-P-methyl-phosphinsäure, P-(3-Aminopropyl)-P-methyl-phosphinsäure und Säureadditionssalzen, Alkalimetallsalzen, dem Magnesiumsalz und dem Ammoniumsalz der P-(3-Aminopropyl)-P-methyl-phosphinsäure.

**2.** Verbindung gemäß Anspruch 1, worin R für Methyl, Fluormethyl, Difluormethyl oder Trifluormethyl steht, $R_1$ für Wasserstoff, Niedrigalkyl, Niedrigalkoxy, Hydroxy, Halogen, Fluormethyl, Difluormethyl oder Trifluormethyl steht und $R_2$ und $R_3$ Wasserstoff bedeuten oder $R_2$ für Hydroxy, Niedrigalkoxy oder Halogen steht und $R_3$ Wasserstoff bedeutet oder $R_2$ und $R_3$ gemeinsam für Oxo stehen.

**3.** Verbindung gemäß Anspruch 1, worin R für Methyl, Fluormethyl, oder Difluormethyl steht, $R_1$ Wasserstoff oder $C_{1-4}$-Alkyl bedeutet, $R_2$ für Wasserstoff oder Hydroxy steht und $R_3$ Wasserstoff bedeutet oder $R_2$ und $R_3$ gemeinsam für Oxo stehen.

**4.** Verbindung gemäß Anspruch 1, worin R für Methyl steht, $R_1$ Wasserstoff darstellt und worin $R_2$ Wasserstoff oder Hydroxy bedeutet und $R_3$ für Wasserstoff steht oder $R_2$ und $R_3$ gemeinsam für Oxo stehen, mit der Maßgabe, daß, wenn $R_2$ für Hydroxy steht, das C-Atom, an das es gebunden ist, die S-Konfiguration aufweist, in der freien Form oder in Form eines Säureadditionssalzes.

**5.** Verbindung gemäß Anspruch 1, worin R für Methyl, Fluormethyl, Difluormethyl oder Trifluormethyl steht, $R_1$ Hydroxy bedeutet und $R_2$ und $R_3$ Wasserstoff darstellen, oder ein Salz hiervon.

**6.** P-[3-Amino-2(R)-hydroxy-propyl]-P-methyl-phosphinsäure oder ein Säureadditions- oder Basensalz hiervon.

**7.** P-[3-Amino-2(S)-hydroxy-propyl]-P-methyl-phosphinsäure oder ein Säureadditions- oder Basensalz hiervon.

**8.** P-(5-Aminopent-3-yl)-P-methyl-phosphinsäure oder ein Säureadditions- oder Basensalz hiervon.

**9.** P-(4-Amino-1,1,1-trifluor-but-2-yl)-P-methyl-phosphinsäure oder ein Säureadditions- oder Basensalz hiervon.

**10.** P-(3-Aminopropyl)-P-fluormethyl-phosphinsäure oder ein Säureadditions- oder Basensalz hiervon.

**11.** P-(3-Aminopropyl)-P-difluormethyl-phosphinsäure oder ein Säureadditions- oder Basensalz hiervon.

**12.** P-(3-Aminopropyl)-P-trifluormethyl-phosphinsäure oder ein Säureadditions- oder Basensalz hiervon.

**13.** Ein Säureadditions- oder Basensalz der P-(3-Amino-2-oxo-propyl)-P-methyl-phosphinsäure.

**14.** P-(4-Aminobut-2-yl)-P-methyl-phosphinsäure oder ein Säureadditions- oder Basensalz hiervon.

**15.** P-(3-Amino-1-hydroxy-propyl)-P-methyl-phosphinsäure oder ein Säureadditions- oder Basensalz hiervon.

**16.** Verbindung für die Verwendung bei einem Verfahren zur Behandlung des menschlichen oder tierischen Körpers, wobei diese Verbindung eine P-substituierte Aminoalkylphosphinsäure der Formel

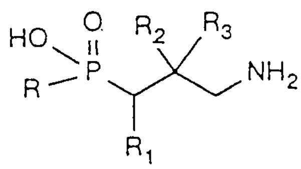

(I),

oder ein pharmazeutisch verträgliches Salz hiervon ist, mit Ausnahme von P-(3-Aminopropyl)-P-methylphosphinsäure und Säureadditionssalzen, Alkalimetallsalzen und des Magnesiumsalzes hiervon, worin R eine gegebenenfalls fluorierte Methylgruppe bezeichnet, $R_1$ für Wasserstoff, Niedrigalkyl, Niedrigalkoxy, Hydroxy, Halogen oder eine fluorierte Methylgruppe steht und $R_2$ und $R_3$ Wasserstoff bedeuten oder $R_2$ Hydroxy, Niedrigalkoxy oder Halogen bezeichnet und $R_3$ für Wasserstoff steht oder $R_2$ und $R_3$ gemeinsam eine Oxogruppe darstellen.

**17.** Verbindung für die Verwendung gemäß Anspruch 16, bei der es sich um P-(3-Aminopropyl)-P-methylphosphinsäure in Form eines pharmazeutisch annehmbaren Ammoniumsalzes hiervon handelt.

**18.** Verbindung für die Verwendung gemäß Anspruch 16, bei der es sich um P-(3-Amino-2-hydroxy-propyl)-P-methyl-phosphinsäure in freier Form oder in Form eines pharmazeutisch annehmbaren Salzes hiervon handelt.

**19.** Verbindung für die Verwendung gemäß Anspruch 16, bei der es sich um P-(3-Amino-2-oxo-propyl)-P-methyl-phosphinsäure in freier Form oder in Form eines pharmazeutisch annehmbaren Salzes hiervon handelt.

**20.** Verbindung gemäß einem der Ansprüche 2 bis 15 für die Verwendung gemäß Anspruch 16.

**21.** Pharmazeutische Zusammensetzung, enthaltend eine Verbindung gemäß einem der Ansprüche 1 bis 20 zusammen mit üblichen pharmazeutischen Trägern.

**22.** Verfahren zur Herstellung von Verbindungen gemäß Anspruch 1, dadurch gekennzeichnet, daß

(a) in einer Verbindung der Formel

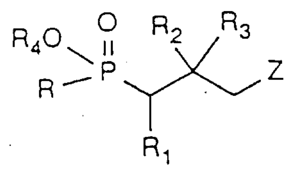

(II),

worin R, $R_1$, $R_2$ und $R_3$ die vorstehend angegebene Bedeutung besitzen, Z für $-NH_2$ steht und $R_4$ eine Hydroxy-Schutzgruppe $R_5$ darstellt oder, wenn R für Methyl steht und $R_1$, $R_2$ und $R_3$ Wasserstoff bedeuten, $R_4$ ein Alkalimetall- oder Ammoniumion $R_6$ ist, oder Z eine geschützte oder latente Aminogruppe $Z_0$ darstellt und $R_4$ für Wasserstoff oder eine Hydroxy-Schutzgruppe $R_5$ steht, und worin eine Carbonylgruppe, die durch $R_2$ und $R_3$ zusammen mit dem Kohlenstoffatom, an das sie gebunden sind, gebildet wird, auch in einer vorübergehend geschützten Form vorliegen kann, jedwede Gruppe $R_5$ oder $R_6$ durch Wasserstoff ausgetauscht wird und/oder jedwede Gruppe $Z_0$ in $-NH_2$ übergeführt wird und/oder, wenn $R_2$ und $R_3$ gemeinsam mit dem Kohlenstoffatom, an das sie gebunden sind, eine geschützte Carbonylgruppe bilden, eine derartige Schutzgruppe entfernt wird; oder

(b) in einer Verbindung der Formel

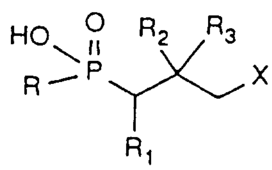

(III),

worin R, $R_1$, $R_2$ und $R_3$ die vorstehend angegebene Bedeutung besitzen und X eine zur Überführung in die Gruppe der Formel $-CH_2-NH_2$ (Ia) befähigte Gruppe darstellt, die Gruppe X in die Gruppe (Ia) übergeführt wird; oder

(c) eine Verbindung der Formel I', die mit einer entsprechenden Verbindung der Formel I mit Ausnahme dessen, daß sie eine oder mehrere Kohlenstoff-Kohlenstoff-Mehrfachbindungen aufweist, identisch ist, zur Bildung einer Verbindung der Formel I reduziert wird, worin R die vorstehend angegebene Bedeutung besitzt, $R_1$ für Wasserstoff, Niedrigalkyl oder fluoriertes Methyl steht und $R_2$ und $R_3$ Wasserstoff sind, und gewünschtenfalls eine entstandene Verbindung in eine andere Verbindung der Formel I übergeführt wird, eine entstandene Mischung der Isomeren in die einzelnen Isomeren getrennt wird und/oder ein entstandenes Salz, das bei diesem Verfahren erhalten wird, in die freie Verbindung der Formel I oder in ein anderes Salz übergeführt wird und/oder gewünschtenfalls eine entstandene freie Verbindung der Formel I in ein Salz entsprechend der vorstehenden Definition übergeführt wird.

**Patentansprüche für folgende Vertragsstaaten : ES, GR**

**1.** Verfahren zur Herstellung einer Verbindung der Formel (I)

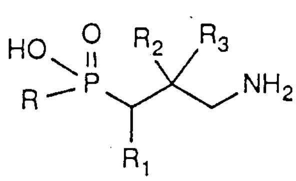

(I),

oder eines Salzes hiervon, worin R für eine gegebenenfalls fluorierte Methylgruppe steht, $R_1$ Wasser-

stoff, Niedrigalkyl, Niedrigalkoxy, Hydroxy, Halogen oder eine fluorierte Methylgruppe bedeutet und $R_2$ und $R_3$ für Wasserstoff stehen oder $R_2$ Hydroxy, Niedrigalkoxy oder Halogen bedeutet und $R_3$ für Wasserstoff steht oder $R_2$ und $R_3$ gemeinsam eine Oxogruppe darstellen mit Ausnahme von P-(3-Amino-2-oxo-propyl)-P-methyl-phosphinsäure, racemischer P-(3-Amino-2-hydroxy-propyl)-P-methyl-phosphinsäure, P-(3-Aminopropyl)-P-methyl-phosphinsäure und Säureadditionssalzen, Alkalimetallsalzen, dem Magnesiumsalz und dem Ammoniumsalz der P-(3-Aminopropyl)-P-methyl-phosphinsäure, dadurch gekennzeichnet, daß

(a) in einer Verbindung der Formel

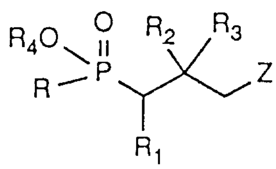

(II),

worin R, $R_1$, $R_2$ und $R_3$ die vorstehend angegebene Bedeutung besitzen, Z für $-NH_2$ steht und $R_4$ eine Hydroxy-Schutzgruppe $R_5$ darstellt oder, wenn R für Methyl steht und $R_1$, $R_2$ und $R_3$ Wasserstoff bedeuten, $R_4$ ein Alkalimetall- oder Ammoniumion $R_6$ ist, oder Z eine geschützte oder latente Aminogruppe $Z_0$ darstellt und $R_4$ für Wasserstoff oder eine Hydroxy-Schutzgruppe $R_5$ steht, und worin eine Carbonylgruppe, die durch $R_2$ und $R_3$ zusammen mit dem Kohlenstoffatom, an das sie gebunden sind, gebildet wird, auch in einer vorübergehend geschützten Form vorliegen kann, jedwede Gruppe $R_5$ oder $R_6$ durch Wasserstoff ausgetauscht wird und/oder jedwede Gruppe $Z_0$ in $-NH_2$ übergeführt wird und/oder, wenn $R_2$ und $R_3$ gemeinsam mit dem Kohlenstoffatom, an das sie gebunden sind, eine geschützte Carbonylgruppe bilden, eine derartige Schutzgruppe entfernt wird; oder

(b) in einer Verbindung der Formel

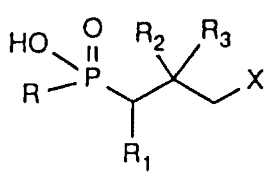

(III),

worin R, $R_1$, $R_2$ und $R_3$ die vorstehend angegebene Bedeutung besitzen und X eine zur Überführung in die Gruppe der Formel $-CH_2-NH_2$ (Ia) befähigte Gruppe darstellt, die Gruppe X in die Gruppe (Ia) übergeführt wird; oder

(c) eine Verbindung der Formel I', die mit einer entsprechenden Verbindung der Formel I mit Ausnahme dessen, daß sie eine oder mehrere Kohlenstoff-Kohlenstoff-Mehrfachbindungen aufweist, identisch ist, zur Bildung einer Verbindung der Formel I reduziert wird, worin R die vorstehend angegebene Bedeutung besitzt, $R_1$ für Wasserstoff, Niedrigalkyl oder fluoriertes Methyl steht und $R_2$ und $R_3$ Wasserstoff sind, und gewünschtenfalls eine entstandene Verbindung in eine andere Verbindung der Formel I übergeführt wird, eine entstandene Mischung der Isomeren in die einzelnen Isomeren getrennt wird und/oder ein entstandenes Salz, das bei diesem Verfahren erhalten wird, in die freie Verbindung der Formel I oder in ein anderes Salz übergeführt wird und/oder gewünschtenfalls eine entstandene freie Verbindung der Formel I in ein Salz entsprechend der vorstehenden Definition übergeführt wird.

**2.** Verfahren gemäß Anspruch 1 zur Herstellung einer Verbindung der Formel (I), worin R für Methyl, Fluormethyl, Difluormethyl oder Trifluormethyl steht, $R_1$ für Wasserstoff, Niedrigalkyl, Niedrigalkoxy, Hydroxy, Halogen, Fluormethyl, Difluormethyl oder Trifluormethyl steht und $R_2$ und $R_3$ Wasserstoff bedeuten oder $R_2$ für Hydroxy, Niedrigalkoxy oder Halogen steht und $R_3$ Wasserstoff bedeutet oder $R_2$ und $R_3$ gemeinsam für Oxo stehen.

3. Verfahren gemäß Anspruch 1 zur Herstellung einer Verbindung der Formel (I), worin R für Methyl, Fluormethyl, Difluormethyl oder Trifluormethyl steht, $R_1$ Wasserstoff oder $C_{1-4}$-Alkyl bedeutet, $R_2$ für Wasserstoff oder Hydroxy steht und $R_3$ Wasserstoff bedeutet oder $R_2$ und $R_3$ gemeinsam für Oxo stehen.

4. Verfahren gemäß Anspruch 1 zur Herstellung einer Verbindung der Formel (I), worin R für Methyl steht, $R_1$ Wasserstoff darstellt und worin $R_2$ Wasserstoff oder Hydroxy bedeutet und $R_3$ für Wasserstoff steht oder $R_2$ und $R_3$ gemeinsam für Oxo stehen, mit der Maßgabe, daß, wenn $R_2$ für Hydroxy steht, das C-Atom, an das es gebunden ist, die S-Konfiguration aufweist, in der freien Form oder in Form eines Säureadditionssalzes.

5. Verfahren gemäß Anspruch 1 zur Herstellung einer Verbindung der Formel I, worin R für Methyl, Fluormethyl, Difluormethyl oder Trifluormethyl steht, $R_1$ Hydroxy bedeutet und $R_2$ und $R_3$ Wasserstoff darstellen, oder eines Salzes hiervon.

6. Verfahren gemäß Anspruch 1 zur Herstellung der P-[3-Amino-2(R)-hydroxy-propyl]-P-methyl-phosphinsäure oder eines Säureadditions- oder Basensalzes hiervon.

7. Verfahren gemäß Anspruch 1 zur Herstellung der P-[3-Amino-2(S)-hydroxy-propyl]-P-methyl-phosphinsäure oder eines Säureadditions- oder Basensalzes hiervon.

8. Verfahren gemäß Anspruch 1 zur Herstellung der P-(5-Aminopent-3-yl)-P-methyl-phosphinsäure oder eines Säureadditions- oder Basensalzes hiervon.

9. Verfahren gemäß Anspruch 1 zur Herstellung der P-(4-Amino-1,1,1-trifluor-but-2-yl)-p-methyl-phosphinsäure oder eines Säureadditions- oder Basensalzes hiervon.

10. Verfahren gemäß Anspruch 1 zur Herstellung der P-(3-Aminopropyl)-p-fluormethyl-phosphinsäure oder eines Säureadditions- oder Basensalzes hiervon.

11. Verfahren gemäß Anspruch 1 zur Herstellung der P-(3-Aminopropyl)-P-difluormethyl-phosphinsäure oder eines Säureadditions- oder Basensalzes hiervon.

12. Verfahren gemäß Anspruch 1 zur Herstellung der P-(3-Aminopropyl)-P-trifluormethyl-phosphinsäure oder eines Säureadditions- oder Basensalzes hiervon.

13. Verfahren gemäß Anspruch 1 zur Herstellung eines Säureadditions- oder Basensalzes der P-(3-Amino-2-oxo-propyl)-P-methyl-phosphinsäure.

14. Verfahren gemäß Anspruch 1 zur Herstellung der P-(4-Aminobut-2-yl)-P-methyl-phosphinsäure oder eines Säureadditions- oder Basensalzes hiervon.

15. Verfahren gemäß Anspruch 1 zur Herstellung der P-(3-Amino-1-hydroxy-propyl)-P-methyl-phosphinsäure oder eines Säureadditions- oder Basensalzes hiervon.

16. Verfahren zur Herstellung einer pharmazeutischen Zusammensetzung, worin eine gemäß einem der Ansprüche 1 bis 15 erhältliche Verbindung mit einem üblichen pharmazeutischen Träger gemischt wird.

17. Verfahren zur Herstellung einer pharmazeutischen Zusammensetzung, worin P-(3-Aminopropyl)-P-methyl-phosphinsäure in Form eines pharmazeutisch annehmbaren Ammoniumsalzes hiervon mit einem üblichen pharmazeutischen Träger gemischt wird.

18. Verfahren zur Herstellung einer pharmazeutischen Zusammensetzung, worin P-(3-Amino-2-hydroxy-propyl)-P-methyl-phosphinsäure in freier Form oder in Form eines pharmazeutisch annehmbaren Salzes hiervon mit einem üblichen pharmazeutischen Träger gemischt wird.

**19.** Verfahren zur Herstellung einer pharmazeutischen Zusammensetzung, worin die P-(3-Amino-2-oxo-propyl)-P-methyl-phosphinsäure in freier Form oder in Form eines pharmazeutisch annehmbaren Salzes hiervon mit einem üblichen pharmazeutischen Träger gemischt wird.

## Revendications

**Revendications pour les Etats contractants suivants : AT, BE, CH, DE, DK, FR, GB, IT, LI, LU, NL, SE**

**1.** Acide aminoalkylphosphinique P-substitué de formule

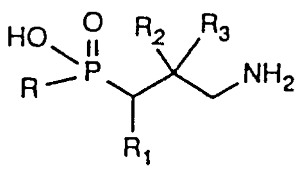

(I),

ou un de ses sels, dans laquelle R représente un groupe méthyle éventuellement fluoré, $R_1$ représente un hydrogène, un alkyle inférieur, un alcoxy inférieur, un hydroxy, un halogène ou un groupe méthyle fluoré et $R_2$ et $R_3$ représentent un hydrogène ou $R_2$ représente un hydroxy, un alcoxy inférieur ou un halogène et $R_3$ est un hydrogène ou $R_2$ et $R_3$ représentent ensemble un groupe oxo, à l'exception de l'acide P-(3-amino-2-oxo-propyl)-P-méthyl-phosphinique, du racémique acide P-(3-amino-2-hydroxypropyl)-P-méthyl-phosphinique,de l'acide P-(3-aminopropyl)-P-méthyl-phosphinique et ses sels d'addition d'acides, ses sels de métal alcalin, le sel de magnésium et le sel d'ammonium de l'acide P-(3-aminopropyl)-P-méthylphosphinique.

**2.** Composé selon la revendication 1, dans laquelle R représente un méthyle, fluorométhyle, difluorométhyle ou trifluorométhyle, $R_1$ représente un hydrogène, un alkyle inférieur, un alcoxy inférieur, un hydroxy, un halogène, un fluorométhyle, un difluorométhyle ou trifluorométhyle et $R_2$ et $R_3$ représentent un hydrogène ou $R_2$ représente un hydroxy, un alcoxy inférieur ou un halogène et $R_3$ représente un hydrogène ou $R_2$ et $R_3$ représentent ensemble un oxo.

**3.** Composé selon la revendication 1, dans laquelle R représente un méthyle, fluorométhyle, difluorométhyle, $R_1$ représente un hydrogène ou un alkyle $C_{1-4}$, $R_2$ représente un hydrogène ou un hydroxy et $R_3$ représente un hydrogène ou $R_2$ et $R_3$ représentent ensemble un oxo.

**4.** Composé selon la revendication 1, dans laquelle R représente un méthyle, $R_1$ est un hydrogène et dans laquelle $R_2$ représente un hydrogène ou un hydroxy et $R_3$ est un hydrogène ou $R_2$ et $R_3$ représentent ensemble un oxo, à la condition que quand $R_2$ représente un hydroxy, l'atome de C auquel il est fixé a la configuration S sous forme libre et sous forme de sel d'addition d'acides.

**5.** Composé de formule I, dans laquelle R représente un méthyle, fluorométhyle, difluorométhyle ou trifluorométhyle, $R_1$ représente un hydroxy et $R_2$ et $R_3$ représentent un hydrogène ou un de ses sels.

**6.** Acide P-[3-amino-2(R)-hydroxy-propyl]-P-méthyl-phosphinique ou un de ses sels d'addition d'acides ou sel de base.

**7.** Acide P-[3-amino-2(S)-hydroxy-propyl]-P-méthyl-phosphinique ou un de ses sels d'addition d'acides ou sel de base.

**8.** Acide P-(5-aminopent-3-yl)-P-méthyl-phosphinique ou un de ses sels d'addition d'acides ou sel de base.

**9.** Acide P-(4-amino-1,1,1-trifluoro-but-2-yl)-P-méthyl-phosphiniqueou un de ses sels d'addition d'acides ou sel de base.

**10.** Acide P-(3-aminopropyl)-P-fluorométhyl-phosphinique ou un de ses sels d'addition d'acides ou sel de base.

**11.** Acide P-(3-aminopropyl)-P-difluorométhyl-phosphinique ou un de ses sels d'addition d'acides ou sel de base.

**12.** Acide P-(3-aminopropyl)-P-trifluorométhyl-phosphinique ou un de ses sels d'addition d'acides ou sel de base.

**13.** Un sel d'addition d'acide ou sel de base de l'acide P-(3-amino-2-oxo-propyl)-P-méthyl-phosphinique.

**14.** Acide P-(4-aminobut-2-yl)-P-méthyl-phosphinique ou un de ses sels d'addition d'acides ou sel de base.

**15.** Acide P-(3-amino-1-hydroxy-propyl)-P-méthyl-phosphinique ou un de ses sels d'addition d'acides ou sel de base.

**16.** Composé pour utilisation dans un procédé de traitement du corps humain ou animal, ledit composé étant un acide aminoalkylphosphinique P-substitué de formule

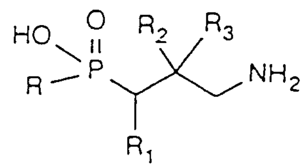

(I),

ou un de ses sels pharmaceutiquement acceptables, à l'exception de l'acide P-(3-aminopropyl)-P-méthyl-phosphinique et ses sels d'addition d'acides, ses sels de métal alcalin, et le sel de magnésium, dans laquelle R représente un groupe utile éventuellement fluoré, $R_1$ représente un hydrogène, un alkyle inférieur, un alcoxy inférieur, un hydroxy, un halogène ou un groupe méthyle fluoré et $R_2$ et $R_3$ représentent un hydrogène ou $R_2$ représente un hydroxy, un alcoxy inférieur ou un halgoène et $R_3$ est un hydrogène ou $R_2$ et $R_3$ représentent ensemble un groupe oxo.

**17.** Composé pour utilisation selon la revendication 16, ledit composé étant l'acide P-(3-aminopropyl)-P-méthyl-phosphonique sous la forme de son sel d'ammonium pharmaceutiquement acceptable.

**18.** Composé pour utilisation selon la revendication 16, ledit composé étant l'acide P-(3-amino-2-hydroxy-propyl)-P-méthyl-phosphinique sous forme libre ou sous forme d'un de ses sels pharmaceutiquement acceptables.

**19.** Composé pour utilisation selon la revendication 16, ledit composé étant l'acide P-(3-amino-2-oxo-propyl)-P-méthyl-phosphinique sous forme libre ou sous la forme d'un de ses sels pharmaceutiquement acceptables.

**20.** Composé selon l'une quelconque des revendications 2 à 15, pour l'utilisation selon la revendication 16.

**21.** Composition pharmaceutique contenant un composé selon l'une quelconque des revendications 1 à 20 en mélange avec des véhicules pharmaceutiques classiques.

**22.** Procédé de préparation de composés selon la revendication 1, caractérisé en ce que :
a) dans un composé de formule

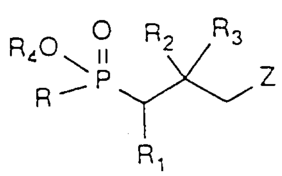

(II),

dans laquelle R, $R_1$, $R_2$ et $R_3$ ont les significations indiquées ci-dessus, Z est $-NH_2$ et $R_4$ est un groupe protecteur d'hydroxy $R_5$ ou, quand R est un méthyle et $R_1$, $R_2$ et $R_3$ représentent l'hydrogène $R_4$ est un métal alcalin ou un ion ammonium $R_6$, ou Z est un groupe amino $Z_0$ latent ou protégé, et $R_4$ est un hydrogène ou un groupe protecteur d'hydroxy $R_5$, et où un groupe carbonyle formé par $R_2$ et $R_3$ ensemble avec l'atome de carbone auquel ils sont attachés peuvent aussi être présents sous une forme de protection temporaire, on remplace l'un quelconque des groupes $R_5$ ou $R_6$ par un hydrogène et/ou on transforme un groupe quelconque $Z_0$ en $-NH_2$ et/ou, si $R_2$ et $R_3$ représentent ensemble avec l'atome de carbone auquel ils sont fixés un groupe carbonyle protégé, on élimine un tel groupe protecteur ; ou

b) dans un composé de formule

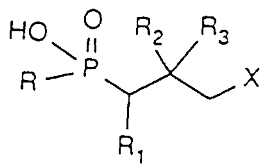

(III),

dans laquelle R, $R_1$, $R_2$ et $R_3$ ont les significations données ci-dessus et X est un groupe pouvant être converti en le groupe de formule $-CH_2-NH_2$ (Ia), on convertit le groupe X en le groupe (Ia) ; ou

c) on réduit un composé de formule I' qui est identique à un composé correspondant de formule I sauf qu'il a une ou plusieurs liaisons multiples carbone-carbone pour donner un composé de formule I dans laquelle R a la signification donnée ci-dessus, $R_1$ est un hydrogène, un alkyle inférieur ou un méthyle fluoré et $R_2$ et $R_3$ représentent un hydrogène et, si c'est souhaité, on convertit un composé résultant en un autre composé de formule I, on fractionne un mélange résultant d'isomère en les isomères individuels et/ou on convertit un sel résultant obtenu dans ce procédé en le composé libre de formule I ou un autre sel et/ou, si c'est souhaité, on convertit un composé libre résultant de formule I en un sel pour correspondre à la définition ci-dessus.

**Revendications pour les Etats contractants suivants : ES, GR**

**1.** Procédé de préparation d'un composé de formule (I)

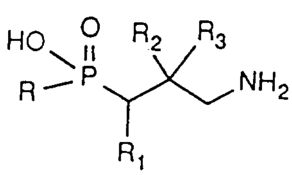

(I),

ou un de ses sels, dans laquelle R représente un groupe méthyle éventuellement fluoré, $R_1$ représente un hydrogène, un alkyle inférieur, un alcoxy inférieur, un hydroxy, un halogène ou un groupe méthyle fluoré et $R_2$ et $R_3$ représentent un hydrogène ou $R_2$ représente un hydroxy, un alcoxy inférieur ou un halogène et $R_3$ est un hydrogène ou $R_2$ et $R_3$ représentent ensemble un groupe oxo, à l'exception de l'acide P-(3-amino-2-oxo-propyl)-P-méthyl-phosphinique, du racémique acide P-(3-amino-2-hydroxypropyl)-P-méthyl-phosphinique,de l'acide P-(3-aminopropyl)-P-méthyl-phosphinique et ses sels d'addition d'acides, ses sels de métal alcalin, le sel de magnésium et le sel d'ammonium de l'acide P-(3-aminopropyl)-P-méthylphosphinique, caractérisé en ce que

a) dans un composé de formule

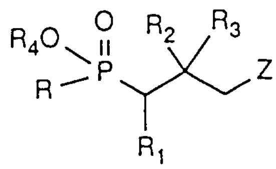

(II),

dans laquelle R, $R_1$, $R_2$ et $R_3$ ont les significations indiquées ci-dessus, Z est $-NH_2$ et $R_4$ est un groupe protecteur d'hydroxy $R_5$ ou, quand R est un méthyle et $R_1$, $R_2$ et $R_3$ représentent l'hydrogène $R_4$ est un métal alcalin ou un ion ammonium $R_6$, ou Z est un groupe amino $Z_0$ latent ou protégé, et $R_4$ est un hydrogène ou un groupe protecteur d'hydroxy $R_5$, et où un groupe carbonyle formé par $R_2$ et $R_3$ ensemble avec l'atome de carbone auquel ils sont attachés peuvent aussi être présents sous une forme de protection temporaire, on remplace l'un quelconque des groupes $R_5$ ou $R_6$ par un hydrogène et/ou on transforme un groupe quelconque $Z_0$ en $-NH_2$ et/ou, si $R_2$ et $R_3$ représentent ensemble avec l'atome de carbone auquel ils sont fixés un groupe carbonyle protégé, on élimine un tel groupe protecteur ; ou

b) dans un composé de formule

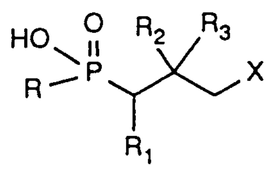

(III),

dans laquelle R, $R_1$, $R_2$ et $R_3$ ont les significations données ci-dessus et X est un groupe pouvant être converti en le groupe de formule $-CH_2-NH_2$ (Ia), on convertit le groupe X en le groupe (Ia) ; ou

c) on réduit un composé de formule I' qui est identique à un composé correspondant de formule I sauf qu'il a une ou plusieurs liaisons multiples carbone-carbone pour donner un composé de formule I dans laquelle R a la signification donnée ci-dessus, $R_1$ est un hydrogène, un alkyle inférieur ou un méthyle fluoré et $R_2$ et $R_3$ représentent un hydrogène et, si c'est souhaité, on convertit un composé résultant en un autre composé de formule I, on fractionne un mélange résultant d'isomère en les isomères individuels et/ou on convertit un sel résultant obtenu dans ce procédé en le composé libre de formule I ou un autre sel et/ou, si c'est souhaité, on convertit un composé libre résultant de formule I en un sel pour correspondre à la définition ci-dessus.

**2.** Procédé selon la revendication 1 de préparation d'un composé de formule (I) dans lequel R représente un méthyle, fluorométhyle, difluorométhyle ou trifluorométhyle, $R_1$ représente un hydrogène, un alkyle inférieur, un alcoxy inférieur, un hydroxy, un halogène, un fluorométhyle, un difluorométhyle ou trifluorométhyle et $R_2$ et $R_3$ représentent un hydrogène ou $R_2$ représente un hydroxy, un alcoxy inférieur ou un halogène et $R_3$ représente un hydrogène ou $R_2$ et $R_3$ représentent ensemble un oxo.

**3.** Procédé selon la revendication de préparation d'un composé de formule (I), dans lequel R représente un méthyle, fluorométhyle, difluorométhyle, $R_1$ représente un hydrogène ou un alkyle $C_{1-4}$, $R_2$ représente un hydrogène ou un hydroxy et $R_3$ représente un hydrogène ou $R_2$ et $R_3$ représentent ensemble un oxo.

**4.** Procédé selon la revendication 1 de préparation d'un composé de formule (I), dans lequel R représente un méthyle, $R_1$ est un hydrogène et dans laquelle $R_2$ représente un hydrogène ou un hydroxy et $R_3$ est un hydrogène ou $R_2$ et $R_3$ représentent ensemble un oxo, à la condition que quand $R_2$ représente un hydroxy, l'atome de C auquel il est fixé a la configuration S sous forme libre et sous forme de sel d'addition d'acides.

**5.** Procédé selon la revendication 1 de préparation d'un composé de formule I, dans lequel R représente un méthyle, fluorométhyle, difluorométhyle ou trifluorométhyle, $R_1$ représente un hydroxy et $R_2$ et $R_3$ représentent un hydrogène ou un de ses sels.

**6.** Procédé selon la revendication 1 de préparation de l'acide P-[3-amino-2(R)-hydroxy-propyl]-P-méthyl-phosphinique ou un de ses sels d'addition d'acides ou sel de base.

**7.** Procédé selon la revendication 1 de préparation de l'acide P-[3-amino-2(S)-hydroxy-propyl]-P-méthyl-phosphinique ou un de ses sels d'addition d'acides ou sel de base.

**8.** Procédé selon la revendication 1 de préparation de l'acide P-(5-aminopent-3-yl)-P-méthyl-phosphinique ou un de ses sels d'addition d'acides ou sel de base.

**9.** Procédé selon la revendication 1 de préparation de l'acide P-(4-amino-1,1,1-trifluoro-but-2-yl)-P-méthyl-phosphinique ou un de ses sels d'addition d'acides ou sel de base.

**10.** Procédé selon la revendication 1 de préparation de l'acide P-(3-aminopropyl)-P-fluorométhyl-phosphinique ou un de ses sels d'addition d'acides ou sel de base.

**11.** Procédé selon la revendication 1 de préparation de l'acide P-(3-aminopropyl)-P-difluorométhyl-phosphinique ou un de ses sels d'addition d'acides ou sel de base.

**12.** Procédé selon la revendication 1 de préparation de l'acide P-(3-aminopropyl)-P-trifluorométhyl-phosphinique ou un de ses sels d'addition d'acides ou sel de base.

**13.** Procédé selon la revendication 1 de préparation d'un sel d'addition d'acide ou sel de base de l'acide P-(3-amino-2-oxo-propyl)-P-méthyl-phosphinique.

**14.** Procédé selon la revendication 1 de préparation de l'acide P-(4-aminobut-2-yl)-P-méthyl-phosphinique ou un de ses sels d'addition d'acides ou sel de base.

**15.** Procédé selon la revendication 1 de préparation de l'acide P-(3-amino-1-hydroxy-propyl)-P-méthyl-phosphinique ou un de ses sels d'addition d'acides ou sel de base.

**16.** Procédé de préparation d'une composition pharmaceutique, dans lequel on mélange un composé obtenu selon l'une quelconque des revendications 1 à 15 à un véhicule pharmaceutique classique.

**17.** Procédé de préparation d'une composition pharmaceutique, dans lequel on mélange l'acide P-(3-amino-propyl)-P-méthyl-phosphinique sous la forme d'un de ses sels d'ammonium pharmaceutiquement acceptables à un véhicule pharmaceutique classique.

**18.** Procédé de préparation d'une composition pharmaceutique, dans lequel on mélange l'acide P-(3-amino-2-hydroxy-propyl)-P-méthyl-phosphinique sous forme libre ou sous forme d'un de ses sels pharmaceutiquement acceptables à un véhicule pharmaceutique classique.

**19.** Procédé de préparation d'une composition pharmaceutique, dans lequel on mélange l'acide P-(3-amino-2-oxo-propyl)-P-méthyl-phosphinique sous forme libre ou sous forme d'un de ses sels pharmaceutiquement acceptables à un véhicule pharmaceutique classique.